# EUROPEAN PATENT SPECIFICATION

(11) **EP 3 061 374 B1**
(45) Date of publication and mention of the grant of the patent: **04.10.2023**
(21) Application number: 16156800.1
(22) Date of filing: 23.02.2016
(51) Int. Cl.: A47K 5/12, A61L 2/00, B05B 11/00, B05B 15/00

(54) **LIQUID DISPENSER WITH REMOVABLE MOBILE DISPENSER**
FLÜSSIGKEITSSPENDER MIT ABNEHMBAREM MOBILEM SPENDER
DISTRIBUTEUR DE LIQUIDE AVEC DISTRIBUTEUR MOBILE AMOVIBLE

(30) Priority: 24.02.2015 CA 2882828; 24.02.2015 CA 2919940
(43) Date of publication of application: 31.08.2016
(60) Divisional application: 23192954.8
(73) Proprietor: OP-Hygiene IP GmbH, 4704 Niederbipp (CH)
(72) Inventor: OPHARDT, Heiner, 4422 Arisdorf (CH); DUNCAN, David R., St. Catharines, Ontario L2N 7E9 (CA)
(74) Representative: Grünecker Patent- und Rechtsanwälte PartG mbB

(56) References cited:
- WO-A1-2010/094963
- GB-A- 2 495 716
- US-A1- 2014 230 960

## Description

### Field of the Invention

This invention relates to devices that dispense hand cleaning fluids, or other liquids. More particularly, the invention relates to a liquid master dispenser having a removable personal or point of care dispenser for providing a mobile supply of the liquid.

### Background of the Invention

The importance of regular hand cleaning in reducing the spread of infectious disease is well known. To encourage frequent hand cleaning, it is becoming increasingly common in health care facilities to incorporate prominent hand cleaning stations at entrances and exits, as well as at other locations throughout. The hand cleaning stations are typically provided with devices for dispensing hand cleaners such as soap or hand sanitizer, which may be manually operated or touchlessly operated.

It is also becoming increasingly common for individuals to carry a supply of hand cleaner in a mobile personal or point of care dispenser, to facilitate hand cleaning when away from a hand cleaning station. This is particularly prevalent in the health care industry, where frequent hand cleaning is essential. Mobile personal or point of care dispensers are often small squeezable containers, which may be deposited in a pocket or clipped to an item of clothing for easy access, or may be placed for use at a point of care.

Despite the increasing awareness of the importance of frequent hand cleaning, the spread of infections in settings such as hospitals and long term care facilities remains problematic. To further combat the spread of infections, some institutions have begun implementing measures for tracking the hand cleaning activity of health care workers. For example, compliance systems may include monitoring hand cleaning and the use of hand cleaners. The present inventors have appreciated that previously known dispensing systems do not provide arrangements for conveniently monitoring the use of cleaners in personal dispensers or for the filling of personal dispensers.

The inventors of this application have appreciated that previously known devices do not integrate the functions of stationary dispensers and mobile dispensers.
Respective dispensers are for example know from US 2014/230960 A1 and WO 2010/094963 A1.

### Summary of the Invention

To at least partially overcome some of the disadvantages of previously known devices, the invention provides a liquid master dispenser that incorporates a removable mobile personal, or point of care, dispenser. The personal dispenser is configured to removably couple to the liquid master dispenser to receive liquid therefrom. The personal dispenser can then be removed and carried to a location remote from the master dispenser, where the liquid in the personal dispenser can be dispensed as needed. In preferred embodiments, the liquid master dispenser is able to dispense the liquid, as for example onto a user's hands, both while the personal dispenser is coupled to the master dispenser and while the personal dispenser is removed from the master dispenser.

The inventors have appreciated that, in at least some embodiments, the liquid dispenser of the invention is able to provide the dual functions of: (i) filling and refilling a mobile personal dispenser; and (ii) directly dispensing liquid, as for example onto a user's hands. In at least some preferred embodiments, the integration of a stationary hand cleaner dispenser with a mobile personal dispenser permits simplified tracking of hand cleaning compliance to take into account the cleaner dispensed from the personal dispenser.
The present invention provides a liquid dispenser comprising the features as claimed in claim 1. Preferred embodiments are the subject of the dependent claims.

In one embodiment, the liquid master dispenser of the invention incorporates a reservoir for containing the liquid to be dispensed, and a dispenser outlet for discharge of the liquid from the reservoir. The liquid master dispenser also incorporates a discharge mechanism, such as a manually or electronically activated pump assembly, which is operable to discharge the liquid from the dispenser outlet. A personal dispenser is removably coupled to the dispenser outlet, and has a receptacle for containing liquid received from the liquid master dispenser. The personal dispenser is provided with an outlet port from which the liquid contained in the receptacle may be discharged.

While the personal dispenser is coupled to the dispenser outlet, the liquid discharged from the dispenser outlet accumulates in the receptacle. As the liquid accumulates, the air displaced thereby may, for example, be expelled from the receptacle via the outlet port or a separate pressure relief valve.

The personal dispenser is configured so that, once the volume of the liquid contained within the receptacle reaches a preselected threshold amount, the receipt of further liquid from the master dispenser causes liquid contained within the personal dispenser to be discharged from the outlet port. This may be achieved, for example, by incorporating a baffle wall into the receptacle which prevents the accumulated liquid from reaching the outlet port until the level of the liquid within the receptacle exceeds the height of the baffle wall. Preferably, the height of the baffle wall is selected so that the receptacle must be nearly full before any liquid is expelled from the outlet port.

The personal dispenser may be removed from the master dispenser at any time, and carried manually to locations remote from the master dispenser. While removed from the master dispenser, the personal dispenser is configured to permit the discharge of liquid contained within the receptacle from the outlet port, to provide a supply of the liquid while away from the master dispenser. This may be achieved, for example, by configuring the receptacle to be manually compressible, so that the liquid is discharged from the outlet port when the personal dispenser is squeezed. While the personal dispenser is removed from the master dispenser, the discharge mechanism of the master dispenser remains operable, so that the liquid contained in the reservoir can be directly discharged from the dispenser outlet.

Furthermore described is a replaceable cartridge for a liquid master dispenser. The replaceable cartridge provides a supply of the liquid to be dispensed, and is configured to be installed in the master dispenser to dispense the liquid therefrom. The cartridge can be removed from the master dispenser and replaced as required. The cartridge includes a reservoir having a reservoir outlet, and a personal dispenser removably coupled to the reservoir outlet. The personal dispenser has a receptacle for containing liquid discharged from the reservoir outlet, and an inlet port for receiving the liquid discharged from the reservoir outlet. An outlet port of the personal dispenser is provided for discharging the liquid from the receptacle.

Preferably, the replaceable cartridge includes a pump assembly disposed across the reservoir outlet that is configured to discharge the liquid from the reservoir upon application of a mechanical force. The mechanical force may be provided, for example, by an actuator carried by a housing of the master dispenser.

Preferably in some embodiments, the personal dispenser is configured to accumulate the liquid received from the reservoir until a preselected volume of the liquid is contained therein, at which point the receipt of further liquid from the reservoir causes discharge of the liquid from the outlet port. The personal dispenser is furthermore removable from the reservoir, and may be carried to locations remote from the master dispenser to selectively discharge the liquid contained therein.

In a further embodiment, the invention provides a refillable personal dispenser for dispensing liquid. The personal dispenser includes a receptacle for containing the liquid, and a coupling mechanism for releasably coupling the personal dispenser to a master dispenser. The personal dispenser also includes an inlet port for receiving liquid discharged from the master dispenser while the personal dispenser is coupled thereto, and an outlet port for discharge of the liquid from the receptacle. The inlet port preferably incorporates a one-way inlet valve that permits fluid to enter the receptacle through the inlet port, and prevents fluid from exiting the receptacle through the inlet port. The outlet port likewise preferably incorporates a one-way outlet valve that permits fluid to exit the receptacle through the outlet port, and prevents fluid from entering the receptacle through the outlet port.

The personal dispenser is preferably configured to accumulate the liquid received from the reservoir until a preselected volume is reached, after which the liquid is discharged from the outlet port when further liquid is received from the reservoir. The personal dispenser is also configured to permit the selective discharge of the liquid contained in the receptacle while uncoupled from the reservoir.

In one embodiment of the invention, a liquid master dispenser is provided that incorporates a reservoir, a dispenser outlet, and a discharge mechanism similar to those described above, and which further includes a separate refill outlet configured to couple with a personal dispenser and deliver liquid from the reservoir to the personal dispenser upon activation of a separate filling mechanism.

Preferably, the liquid dispenser incorporates a usage monitoring system which records the amount of liquid that has been dispensed from both the dispenser outlet and the refill outlet. This ensures that the usage monitoring system takes into account the liquid that is used to fill the personal dispenser, and not just the liquid that is directly dispensed from the dispenser outlet. As such, the usage monitoring system is able to create a more complete record of liquid usage, which may for example be particularly useful in monitoring hand cleaning compliance in a health care setting.

Accordingly, in one aspect the present invention resides in a liquid master dispenser, comprising:
a reservoir for containing liquid to be dispensed;
a dispenser outlet for discharge of the liquid from the reservoir;
a discharge mechanism operable to discharge the liquid from the dispenser outlet when activated; and
a personal dispenser removably coupled to the dispenser outlet, the personal dispenser comprising a receptacle for containing the liquid and an outlet port for discharge of the liquid from the receptacle;
wherein:
   (a) while the personal dispenser is coupled to the dispenser outlet, the liquid discharged from the dispenser outlet accumulates in the receptacle until the receptacle contains a preselected volume of the liquid, and once the receptacle contains the preselected volume of the liquid, the discharge of further said liquid from the dispenser outlet causes the liquid contained in the receptacle to be discharged from the outlet port; and
   (b) while the personal dispenser is removed from the dispenser outlet:
      (i) the personal dispenser is configured to be carried manually to locations remote from the master dispenser for selectively discharging the liquid contained in the receptacle from the outlet port; and
      (ii) the discharge mechanism is operable to discharge the liquid contained in the reservoir from the dispenser outlet.

In another aspect, the present invention resides in a replaceable cartridge for a liquid master dispenser, comprising:
a reservoir for containing liquid to be dispensed, the reservoir having a reservoir outlet for selective discharge of the liquid from the reservoir; and
a personal dispenser removably coupled to the reservoir outlet, the personal dispenser comprising:
a receptacle for containing the liquid discharged from the reservoir outlet;
an inlet port for receiving the liquid discharged from the reservoir outlet and communicating the liquid to the receptacle; and
an outlet port for discharge of the liquid from the receptacle;
wherein the reservoir outlet is configured to permit the selective discharge of the liquid from the reservoir while the personal dispenser is removed from the reservoir outlet;
wherein the personal dispenser is configured so that, while the personal dispenser is coupled to the reservoir outlet, the liquid discharged from the reservoir outlet accumulates in the receptacle until the receptacle contains a preselected volume of the liquid, and once the receptacle contains the preselected volume of the liquid, the discharge of further said liquid from the reservoir outlet causes the liquid contained in the receptacle to be discharged from the outlet port; and
wherein the personal dispenser is configured for selectively discharging the liquid contained in the receptacle from the outlet port, after the personal dispenser is removed from the reservoir outlet.

In a further aspect, the present invention resides in a refillable personal dispenser for dispensing liquid, comprising:
a receptacle for containing the liquid;
a coupling mechanism for releasably coupling to a reservoir;
an inlet port for receiving the liquid from the reservoir and communicating the liquid to the receptacle; and
an outlet port for discharge of the liquid from the receptacle;
wherein the outlet port is configured so that, while the personal dispenser is coupled to the reservoir, the liquid received by the inlet port accumulates in the receptacle until the receptacle contains a preselected volume of the liquid; and once the receptacle contains the preselected volume of the liquid, the outlet port is configured to discharge the liquid contained in the receptacle upon receipt of further said liquid by the inlet port;
wherein the inlet port comprises a one-way inlet valve that permits fluid to enter the receptacle through the inlet port, and prevents fluid from exiting the receptacle through the inlet port; and
wherein the receptacle defines an internal volume and is compressible to selectively reduce the internal volume from an uncompressed volume to a compressed volume upon application of a compressing force, to thereby discharge the liquid from the outlet port.

In a still further aspect, the present invention resides in a liquid dispenser, comprising:
a reservoir for containing liquid to be dispensed;
a dispenser outlet for discharge of the liquid from the reservoir;
a discharge mechanism operable to discharge the liquid from the dispenser outlet when activated;
a refill outlet configured to couple with a personal dispenser and deliver the liquid from the reservoir to the personal dispenser; and
a filling mechanism operable to deliver the liquid from the refill outlet when activated.

In yet another aspect, the present invention resides in a hand cleaning liquid master dispenser, comprising:
a reservoir for containing liquid to be dispensed;
a discharge mechanism operable to discharge the liquid from the reservoir when activated; and
a personal dispenser removably coupled to the master dispenser, the personal dispenser comprising a receptacle for containing the liquid and an outlet port for discharge of the liquid from the receptacle;
wherein:
   (a) while the personal dispenser is coupled to the master dispenser, the discharge mechanism discharges the liquid from the reservoir into the receptacle and the liquid discharged accumulates in the receptacle until the receptacle contains a preselected volume of the liquid, and while the receptacle contains the preselected volume of the liquid, the discharge mechanism discharges the liquid from a dispenser outlet; and
   (b) while the personal dispenser is removed from the master dispenser:
      (i) the personal dispenser is configured to be carried manually to locations remote from the master dispenser for selectively discharging the liquid contained in the receptacle from the outlet port; and
      (ii) the discharge mechanism is operable to discharge the liquid contained in the reservoir from the dispenser outlet.

### Brief Description of the Drawings

Further aspects and advantages of the invention will appear from the following description taken together with the accompanying drawings, in which:
Figure 1 shows a perspective view of a liquid master dispenser in accordance with a first preferred embodiment of the invention;
Figure 2A shows a pictorial side view of the liquid master dispenser of Figure 1 with the housing removed, and a personal dispenser in a coupled state;
Figure 2B show a pictorial side view of the liquid master dispenser as in Figure 2A but with the personal dispenser in an uncoupled state supported on an independent desktop support surface;
Figure 2C shows a cross-sectional view Figure 2A showing the liquid master dispenser and the personal dispenser in the coupled state;
Figure 3 shows a perspective view of the personal dispenser of the liquid master dispenser of Figure 1;
Figure 4 shows a top view of the personal dispenser of Figure 3;
Figure 5 shows a cross sectional view of the personal dispenser of Figure 4, taken along line A-A';
Figure 6 shows a perspective view of a mounting dock of the liquid master dispenser of Figure 2A;
Figure 7 shows a bottom view of the mounting dock of Figure 6;
Figures 8 to 10 are perspective views depicting the coupling of the personal dispenser of the liquid master dispenser of Figure 1, and showing successive relative positions moving from an initial coupling position of Figure 8, through an intermediate position of Figure 9, to a fully coupled position of Figure 10;
Figure 11 shows a rear view of the personal dispenser coupled to the mounting dock taken along section line B-B' in Figure 2A;
Figure 12 shows a cross sectional view of a second embodiment of a personal dispenser similar to that shown in Figure 5, with a first alternate construction;
Figure 13 shows a cross sectional view of a third embodiment of a personal dispenser similar to that shown in Figure5, with a second alternate construction;
Figure 14 shows a perspective view of a liquid master dispenser in accordance with a second preferred embodiment of the invention with a personal dispenser in an uncoupled state;
Figure 15 is a schematic diagram of a control circuit for the dispenser of Figure 14;
Figure 16 shows a front view of a liquid master dispenser in accordance with a third preferred embodiment of the invention with a personal dispenser in a coupled state;
Figure 17 shows a front view of Figure 16 with the personal dispenser in an uncoupled state;
Figure 18 shows a front view of a replaceable cartridge of the liquid master dispenser of Figure 16 with the personal dispenser in a coupled state;
Figure 19 shows a cross-sectional front view of a pump assembly of the replaceable cartridge of Figure 18;
Figure 20 shows a perspective view of a personal dispenser of the replaceable cartridge of Figure 16;
Figure 21 shows a cross-sectional front view of the personal dispenser of Figure 20;
Figure 22 shows a schematic front view of the pump assembly, an actuator plate, and the personal dispenser of the dispenser of Figure 16;
Figure 23 shows a perspective view of a liquid master dispenser in accordance with a fourth preferred embodiment of the invention;
Figure 24 shows a pictorial side view of the liquid master dispenser of Figure 23 with the housing removed, and a personal dispenser in a coupled state to a refill tube;
Figure 25 shows an enlarged vertical cross-sectional view of the valve assembly in Figure 24 in a diversion condition as in Figure 24 to direct fluid via the refill tube to the personal dispenser;
Figure 26 shows the enlarged vertical cross-sectional view of the valve assembly as in Figure 25 but in a flow through condition as in Figure 23 to direct fluid to a discharge outlet;
Figure 27 shows a perspective view of a reservoir cartridge, comprising a reservoir with a personal dispenser held in a cavity thereof, in accordance with a fifth preferred embodiment of the invention;
Figure 28 shows a perspective view of the reservoir and personal dispenser of Figure 27, with the personal dispenser removed from the cavity;
Figure 29 shows a side view of a liquid master dispenser in accordance with a sixth preferred embodiment of the invention, with a personal dispenser in an uncoupled state;
Figure 30 shows a side view of the liquid master dispenser of Figure 29, with the personal dispenser coupled to a spout tube of the master dispenser;
Figure 31 shows a perspective view of a personal dispenser in accordance with a seventh preferred embodiment of the invention;
Figure 32 shows a side view of a liquid master dispenser incorporating the personal dispenser of claim 31, with the personal dispenser in an uncoupled state;
Figure 33 shows a side view of the liquid master dispenser of Figure 32, with the personal dispenser in a coupled state;
Figure 34 shows a cross-sectional view of the personal dispenser of Figure 31 along section line C-C';
Figure 35 shows a cross-sectional view of the personal dispenser of Figure 31, which is the same view as shown in Figure 34, but with the personal dispenser in a coupled state as in Figure 33;
Figure 36 shows a cross-sectional perspective view of the personal dispenser of Figure 32 along sectional line E-E', with the personal dispenser in the uncoupled state; and
Figure 37 shows a cross-sectional perspective view of the personal dispenser of Figure 33 along sectional line F-F', with the personal dispenser in the coupled state.

### Detailed Description of the Drawings

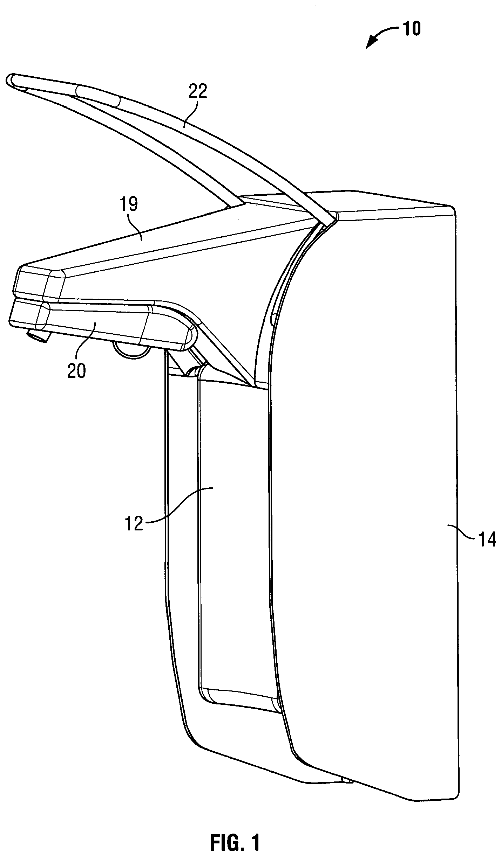

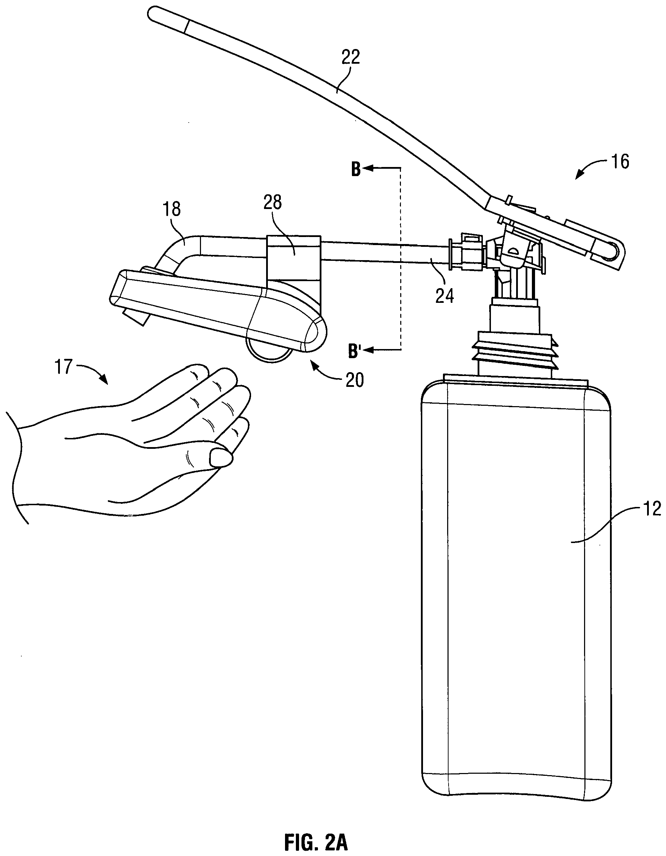

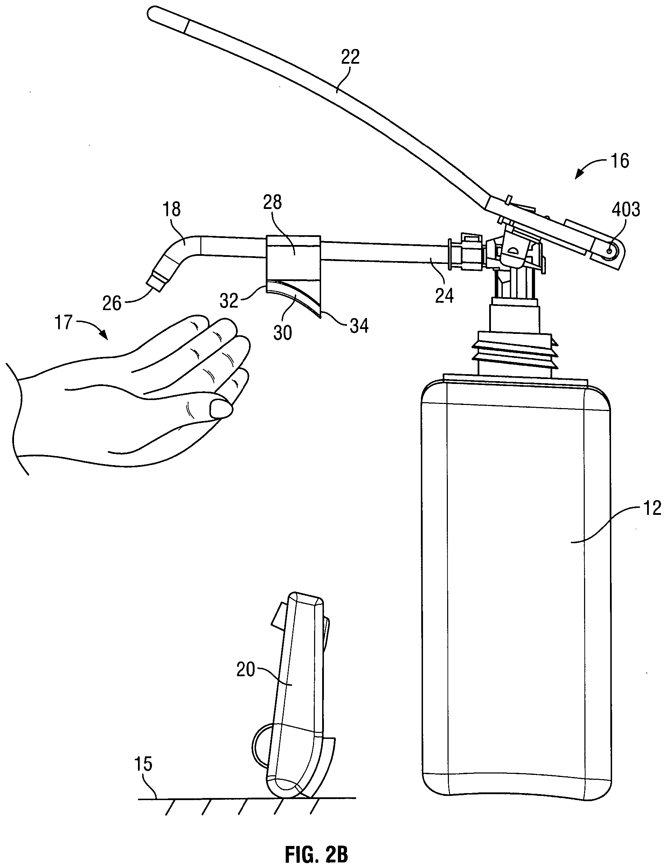

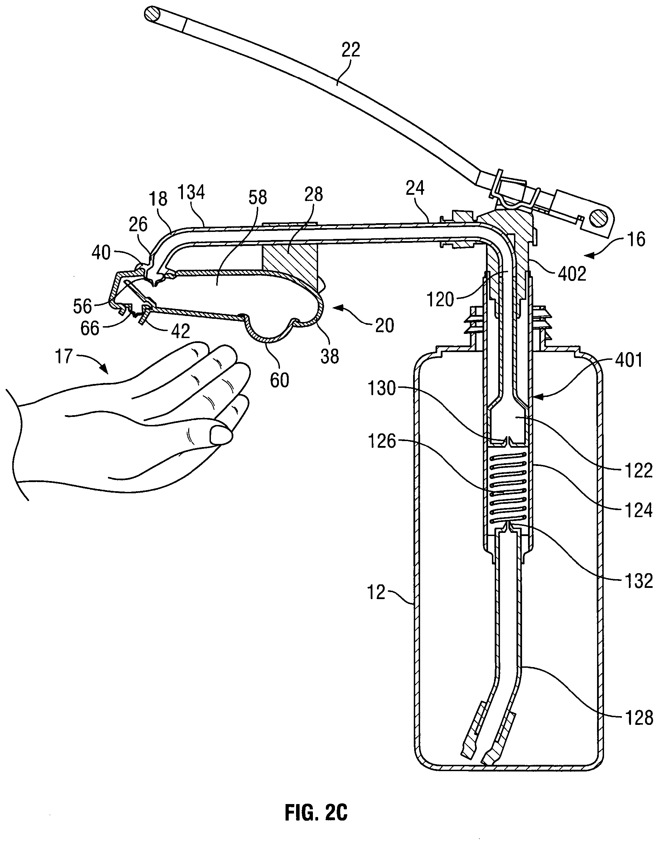

Reference is made first to Figures 1, 2A, 2B and 2C which show a liquid master dispenser 10 in accordance with a first preferred embodiment of the invention. The master dispenser 10 includes a reservoir 12, a housing 14, a pump assembly 16, a dispenser outlet 18, and a personal dispenser or point of care dispenser 20. The master dispenser 10 is similar to that disclosed in U.S. Patent No. 7,748,573 to Ophardt et al., issued July 6, 2010, and in U.S. Publication No. US 2014/025336 to Ophardt.

The reservoir 12 is a container for holding the liquid to be dispensed from the master dispenser 10. The housing 14 partially surrounds and supports the reservoir 12, the pump assembly 16, and the dispenser outlet 18. The housing 14 is adapted for permanent attachment to a wall or other similar structure, although the housing 14 could alternatively be free standing as on a table top or other support surface, or selectively removable from a stand or other support. The housing 14 carries a discharge tube shroud 19.

The pump assembly 16 is configured to pump the liquid from the reservoir 12 and out through the dispenser outlet 18 upon application of a mechanical force thereto. In the preferred embodiment shown, a manual actuator 22 is provided for applying the mechanical force to the pump assembly 16.

The pump assembly 16 is best shown in Figure 2C as including a piston chamber forming body 401 and a piston forming element 402. The piston chamber forming body 401 is fixed to the housing 14 against movement and defines therein a piston chamber 124 from which a dip tube 128 extends downwardly in to the reservoir 12. The reservoir 12 is supported by the housing 14. The piston forming element 402 is mounted to the piston chamber forming body 401 for relative vertical movement with a piston 122 of the piston forming element 402 coaxially slidable within piston chamber 124 biased upwardly by a spring 126 disposed within the piston chamber 124 between the piston chamber 124 and the piston 122. A manual actuator 22 in the form of a pivoting lever is mounted to the housing 14 for pivoting about a horizontal axis to move the piston forming element 402 downwardly relative to the piston chamber forming body 401 against the bias of the spring 126.

The piston forming element 402 includes a hollow spout tube 134 that extends from the piston 122 forming an internal conduit 120 therein that is integral with a dispenser outlet 18. At one end, the internal conduit 120 expands radially outward to form the piston 122, which sits snugly within the piston chamber 124. The piston 122 is provided with a duckbill check valve 130, which permits fluid to flow into the piston 122 from the piston chamber 124, and prevents fluid from flowing out of the piston 122 into the piston chamber 124.

The piston chamber 124 defines a cylindrical cavity within which the piston 122 is able to move up and down. The spring 126 sits within the piston chamber 124 below the piston 122, and biases the piston 122 upwards. The dip tube 128 extends downwardly from the piston chamber 124 toward the bottom of the reservoir 12, for drawing the liquid therefrom. A one-way duckbill valve 132 sits between the piston chamber 124 and the dip tube 128, and permits fluid to flow into the piston chamber 124 from the dip tube 128, and prevents fluid from flowing out of the piston chamber 124 into the dip tube 128.

The pump assembly 16 is operated by depressing the manual actuator 22, which causes the internal conduit 120 and the piston 122 to slide downwardly within the piston chamber 124. The movement of the piston 122 pressurizes the fluid contained within the piston chamber 124, forcing the fluid upwards through the check valve 130. The influx of fluid into the piston 122 displaces any fluid contained therein, which in turn displaces any fluid contained within the internal conduit 120 and forces the fluid out through the dispenser outlet 18.

When the manual actuator 22 is released, the spring 126 pushes the piston 122 back up to its initial position. This reduces the pressure within the piston chamber 124, which draws liquid in from the reservoir 12 via the dip tube 128 and through the valve 132. The process may then be repeated as desired.

Initially, the pump assembly 16 is primed by operating the pump assembly 16 repeatedly until all of the air contained therein is completely displaced by liquid from the reservoir 12. Once the pump assembly 16 is fully primed, further operation of the pump assembly 16 will force the liquid contained therein out through the dispenser outlet 18.

As shown in Figure 2C, the dispenser outlet 18 is an outer portion of the hollow tubular elongated spout tube 134 that extends horizontally from the internal conduit 120, and is in fluid communication with the reservoir 12 via the pump assembly 16 for receiving the liquid therefrom. The spout tube 134 extends from an inlet end 24 that is continuous with the internal conduit 120, to an outlet end 26 from which the liquid is dispensed. The spout tube 134 curves downward near the outlet end 26, so that the outlet end 26 is angled below horizontal, that is extends forwardly as it extends downwardly, to direct the liquid into a user's hand 17 disposed below the outlet end 26 during dispensing.

A mounting dock 28 is coupled to the spout tube 134 between the inlet end 24 and the outlet end 26. The mounting dock 28 is provided for sliding engagement with the personal dispenser 20, to enable coupling and uncoupling of the personal dispenser 20 with the spout tube 134.

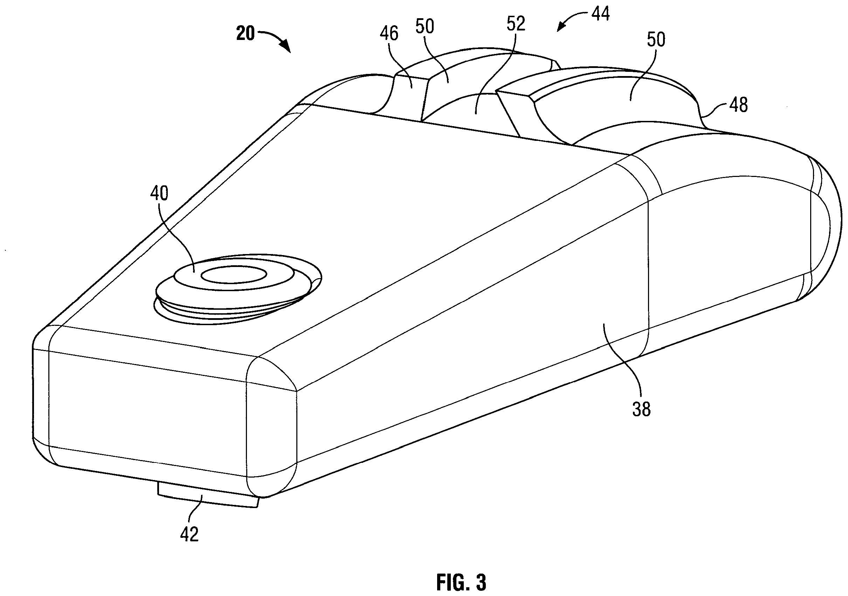

The personal dispenser 20 is best shown in Figures 3, 4 and 5 as including a receptacle 38, an inlet port 40, an outlet port 42, and a generally arcuate docking seat 44. The inlet port 40 is configured to receive the liquid discharged from the dispenser outlet 18, and is provided with a one-way inlet valve 56 that permits fluid to enter the receptacle 38 through the inlet port 40, and prevents fluid from exiting the receptacle 38 through the inlet port 40. The one-way inlet valve 56 is a duckbill valve, although any suitable one-way valve construction could be employed.

The inlet port 40 is configured so that, while the personal dispenser 20 is coupled to the spout tube 134, the inlet port 40 sealingly engages with the outlet end 26 of the spout tube 134, as best seen in Figure 2C. This ensures that the liquid discharged from the outlet end 26 passes through the one-way inlet valve 56 and into the receptacle 38.

The receptacle 38 is configured to contain the liquid received by the inlet port 40, and defines an internal cavity 58 within which the liquid received from the inlet port 40 accumulates. The receptacle 38 incorporates a dispensing mechanism for selectively dispensing the liquid contained therein. In the embodiment shown, the dispensing mechanism is a manually compressible diaphragm pump 60.

The diaphragm pump 60 is formed as an outwardly bulging semi-spherical membrane, preferably constructed from a resiliently flexible plastic or rubber material. The diaphragm pump 60 is configured to be inwardly compressible upon application of a manual compressing force, to selectively reduce the internal volume of the cavity 58 from an uncompressed volume to a compressed volume, to thereby discharge the liquid contained in the receptacle 38 through the outlet port 42. The diaphragm pump 60 is biased toward returning to the outwardly bulging semi-spherical shape upon release of the compressing force, to return the internal volume of the cavity 58 to its uncompressed volume.

The receptacle 38 also incorporates a baffle 62 that is interposed between the inlet port 40 and the outlet port 42. The baffle 62 forms a partial barrier between the outlet port 42 and the internal cavity 58, leaving only a narrow upper channel 64 permitting fluid communication therebetween. The configuration of the baffle 62 permits the expulsion of displaced air from the receptacle 38 as the liquid accumulates therein.

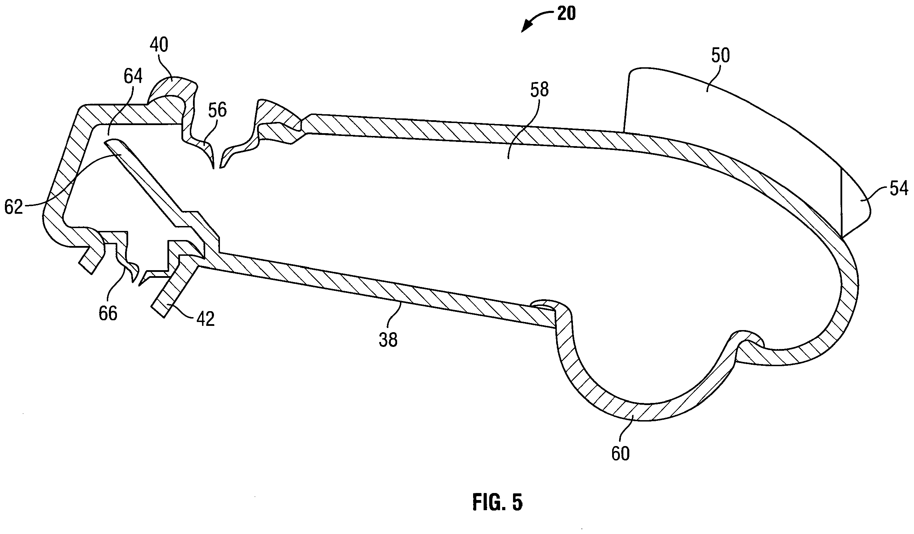

The outlet port 42 is configured to discharge the liquid from the receptacle 38, and is provided with a one-way outlet valve 66 that permits fluid to exit the receptacle 38 through the outlet port 42, and prevents fluid from entering the receptacle 38 through the outlet port 42. In the embodiment shown in Figure 5, the one-way outlet valve 66 is a duckbill valve, although any suitable one-way valve construction could be employed. Preferably, the one-way outlet valve 66 is configured to discharge fluid from the receptacle 38 only when a pressure differential across the outlet port 42 exceeds a preselected threshold. This helps to ensure that the liquid does not unintentionally leak out of the personal dispenser 20. The preselected threshold is preferably between 50 mbar and 100 mbar, and most preferably at least about 60 mbar.

The operation of the master dispenser 10 while the personal dispenser 20 is coupled thereto will now be described. To fill the personal dispenser 20 with the liquid, the pump assembly 16 is operated to discharge the liquid from the dispenser outlet 18 in the manner previously described. The liquid discharged by the dispenser outlet 18 is received by the inlet port 40, where it passes through the one-way inlet valve 56 and into the receptacle 38.

While in the coupled orientation shown in Figure 2C, the personal dispenser 20 is oriented such that the received liquid initially pools within the internal cavity 58 below the height of the baffle 62. Meanwhile, the lower relative density of the air contained within the internal cavity 58 causes the air to sit above the liquid, in fluid communication with the outlet port 42 through the upper channel 64. As the liquid accumulates, the pressure within the internal cavity 58 rises until the preselected threshold of the one-way outlet valve 66 is reached. Once the preselected threshold is reached, the one-way outlet valve 66 opens to release the displaced air therefrom.

Eventually, the level of the liquid contained within the internal cavity 58 reaches the height of the baffle 62. At this point, most of the air initially contained within the internal cavity 58 has been expelled from the outlet port 42. As further liquid is received by the inlet port 40, the liquid spills over the baffle 62 through the upper channel 64 to the outlet port 42, to be discharged when the pressure is above the preselected threshold.

The construction of the liquid master dispenser 10 permits the liquid to be discharged while the personal dispenser 20 remains coupled to the dispenser outlet 18. In particular, once the level of liquid within the internal cavity 58 has exceeded the height of the baffle 62, allowing the liquid to pool above the outlet port 42, further pressurization of the internal cavity 58 above the preselected threshold will cause the discharge of the liquid from the outlet port 42.

One way to further pressurize the internal cavity 58 is by activating the pump assembly 16, to introduce further liquid into the receptacle 38 through the inlet port 40. This influx of liquid will increase the pressure within the internal cavity 58. Once the preselected threshold is reached, the liquid pooled above the outlet port 42 will be discharge therefrom.

The personal dispenser 20 can be easily uncoupled from the dispenser outlet 18 when desired, and is configured to sit upright on a flat support surface 15 such as a table or desk, as shown in Figure 2B. When detached from the dispenser outlet 18, the liquid is discharged from the personal dispenser 20 by manually compressing the diaphragm pump 60. This pressurizes the cavity 58, to thereby open the one-way outlet valve 66 and discharge the liquid therefrom. The one-way inlet valve 56 furthermore allows air to enter the receptacle 38 through the inlet port 40, to replace the discharged liquid and permit the diaphragm pump 60 to return to its original outwardly bulging semi-spherical shape. The diaphragm pump 60 can then be compressed again to discharge further liquid as desired.

While the personal dispenser 20 is removed from the dispenser outlet 18, the pump assembly 16 remains operable to dispense the liquid directly from the dispenser outlet 18, as for example onto a person's hand below the outlet end 26, as shown in Figure 2B.

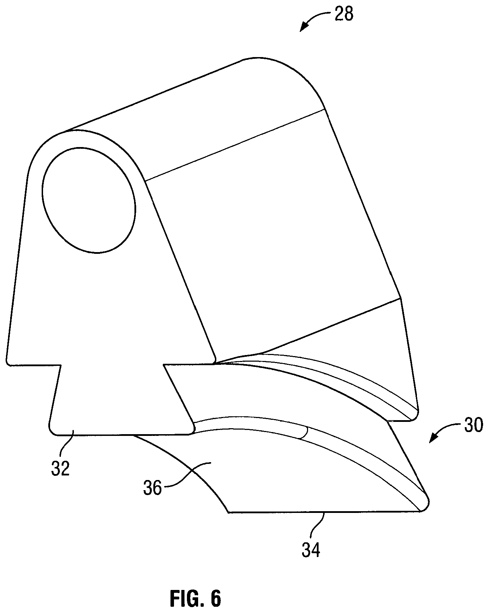

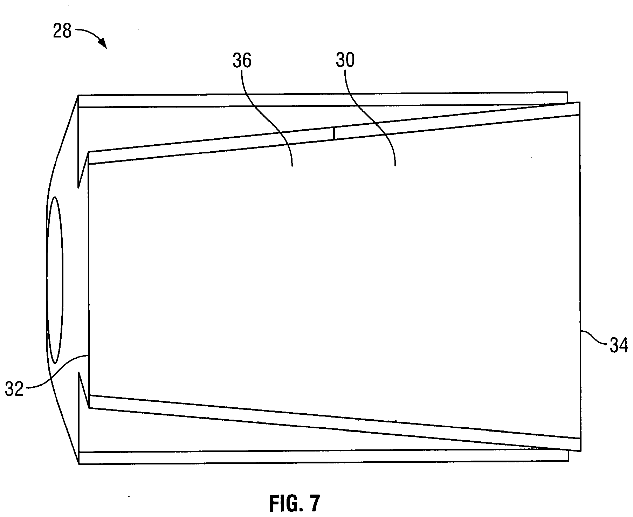

The structures which permit the coupling of the personal dispenser 20 to the dispenser outlet 18 will now be described in detail. As best shown in Figure 6, the mounting dock 28 defines a generally arcuate track 30 that extends from a front end 32 to a back end 34. The front end 32 of the generally arcuate track 30 faces the outlet end 26 of the spout tube 134, and the back end 34 faces away from the outlet end 26, as shown in Figure 2B. The generally arcuate track 30 is formed as an elongated dovetail shaped projection 36. The elongated dovetail shaped projection 36 is tapered such that a width of the elongated dovetail shaped projection 36 at the front end 32 of the generally arcuate track 30 is less than a width of the elongated dovetail shaped projection 36 at the back end 34 of the generally arcuate track 30, as is best shown in Figure 7.

The generally arcuate docking seat 44 of the personal dispenser 20 is provided for sliding engagement with the generally arcuate track 30 of the mounting dock 28, to enable coupling and uncoupling of the personal dispenser 20 and the dispenser outlet 18. As shown in Figure 3, the generally arcuate docking seat 44 extends from a front end 46 that faces the inlet port 40, to a back end 48 that faces away from the inlet port 40, and is formed from two docking rails 50. Together, the docking rails 50 define an elongated dovetail shaped channel 52 therebetween, which is configured to receive the elongated dovetail shaped projection 36.

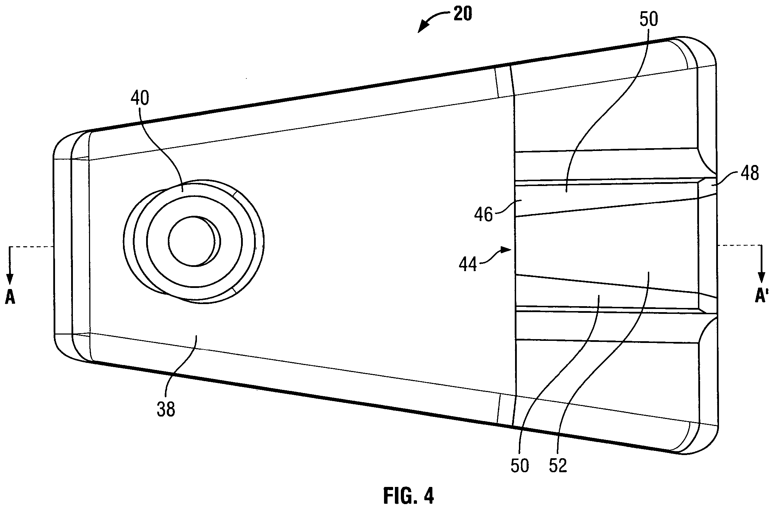

As is best shown in Figure 4, the docking rails 50 define a taper in the elongated dovetail shaped channel 52, such that a width of the elongated dovetail shaped channel 52 at the front end 46 of the generally arcuate docking seat 44 is less than a width of the elongated dovetail shaped channel 52 at the back end 48 of the generally arcuate docking seat 44. More particularly, the taper in the elongated dovetail shaped channel 52 is selected to correspond to the taper in the elongated dovetail shaped projection 36, such that the elongated dovetail shaped channel 52 and the elongated dovetail shaped projection 36 fit snugly together when the elongated dovetail shaped projection 36 is fully received within the elongated dovetail shaped channel 52.

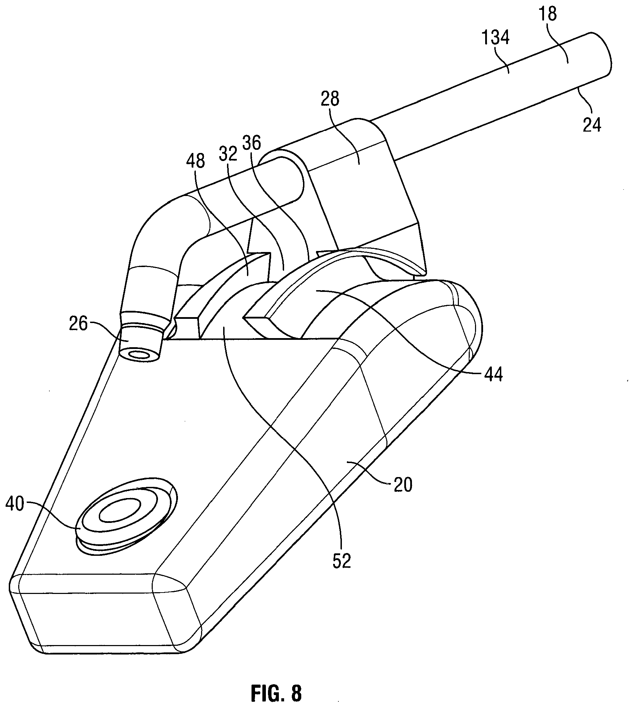

The manner by which the personal dispenser 20 is coupled with the dispenser outlet 18 will now be described with reference to Figures 8 to 10. As shown in Figure 8, the front end 32 of the elongated dovetail shaped projection 36 is initially received within the back end 48 of the elongated dovetail shaped channel 52. The arrangement of the generally arcuate docking seat 44 on the personal dispenser 20 is selected such that the inlet port 40 of the personal dispenser 20 is spaced from the outlet end 26 of the spout tube 134 in this initial position.

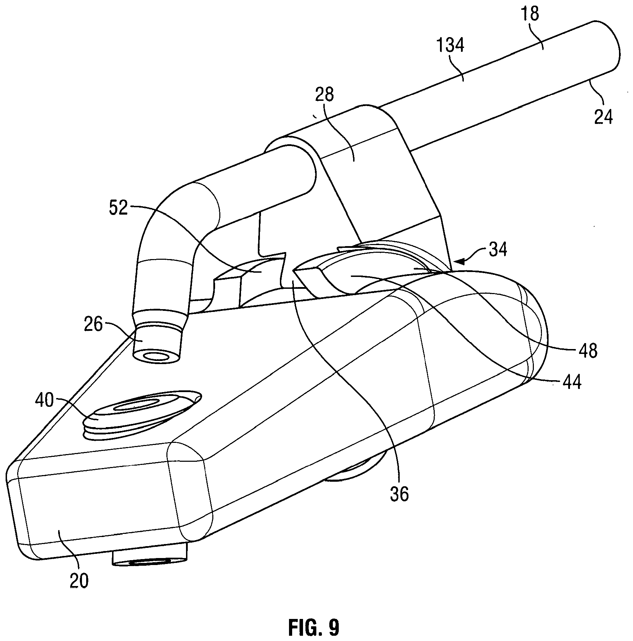

Next, the back end 48 of the generally arcuate docking seat 44 is slid along the elongated dovetail shaped projection 36 toward the back end 34 thereof. At the same time, the curvature of the elongated dovetail shaped channel 52 and the elongated dovetail shaped projection 36 cause the inlet port 40 to move toward the outlet end 26 of the spout tube 134, as shown in Figure 9.

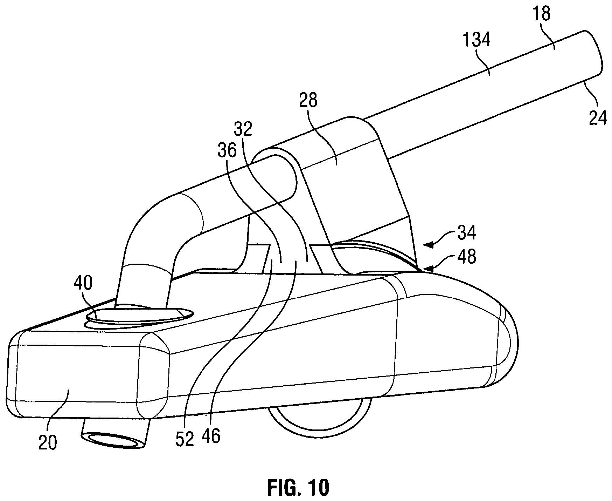

The elongated dovetail shaped channel 52 and the elongated dovetail shaped projection 36 are fully engaged once the front end 32 of the elongated dovetail shaped projection 36 is engaged with the front end 46 of the elongated dovetail shaped channel 52, and the back end 34 of the elongated dovetail shaped projection 36 is engaged with the back end 48 of the elongated dovetail shaped channel 52, as shown in Figure 10. At this point, the outlet end 26 of the spout tube 134 is received within the inlet port 40 of the personal dispenser 20.

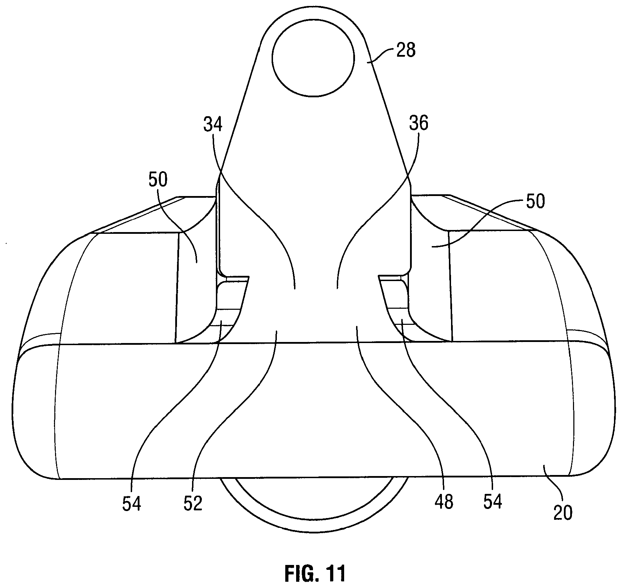

The corresponding tapers in the elongated dovetail shaped channel 52 and the elongated dovetail shaped projection 36 prevent the front end 46 of the elongated dovetail shaped channel 52 from sliding past the front end 32 of the elongated dovetail shaped projection 36 toward the back end 34 of the elongated dovetail shaped projection 36. As is best shown in Figure 11, the docking rails 50 are each provided with a flexible retaining boss 54 at the back end 48 of the elongated dovetail shaped channel 52. The flexible retaining bosses 54 are configured to sit behind the elongated dovetail shaped projection 36 in a snap fit while the elongated dovetail shaped projection 36 is fully engaged with the elongated dovetail shaped channel 52, to provide a preselected resistance against the sliding removal of the elongated dovetail shaped projection 36 from the elongated dovetail shaped channel 52. A skilled artisan will appreciate that by adjusting the relative dimensions and material properties of the flexible retaining bosses 54, the degree of resistance can be adjusted. Preferably, the degree of resistance is selected so as to prevent unintentional disengagement of the elongated dovetail shaped projection 36 from the elongated dovetail shaped channel 52, while also permitting the personal dispenser 20 to be easily removed from the mounting dock 28 when so desired.

The personal dispenser 20 is removed from the mounting dock 28 by sliding the back end 48 of the elongated dovetail shaped channel 52 toward the front end 32 of the elongated dovetail shaped projection 36, until the elongated dovetail shaped channel 52 and the elongated dovetail shaped projection 36 are completely disengaged. At the same time, the curvature of the elongated dovetail shaped channel 52 and the elongated dovetail shaped projection 36 cause the inlet port 40 of the personal dispenser 20 to disengage from the outlet end 26 of the dispenser outlet 18.

The interaction of the mounting dock 28 and the docking seat 44 on the personal dispenser 20 provides one arrangement for pivotal movement of the personal dispenser 20 into engagement with the discharge outlet. Other arrangements may be used.

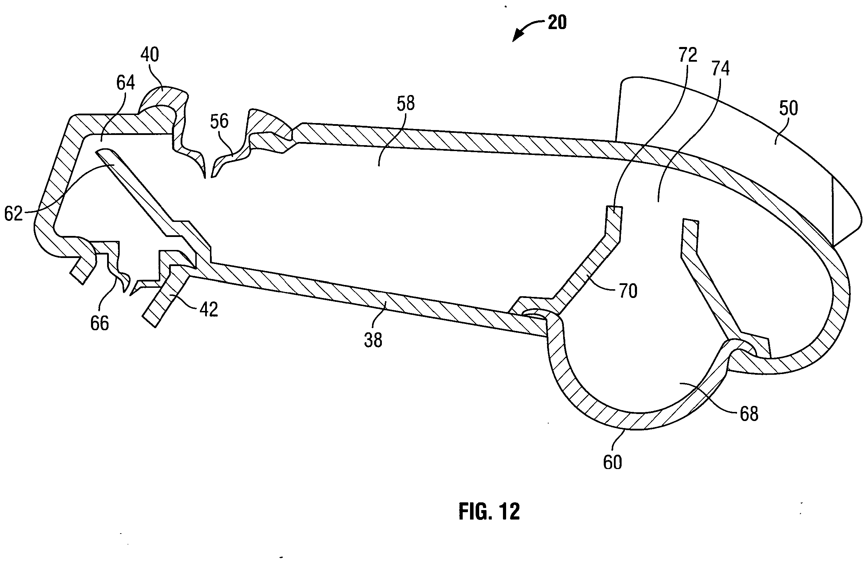

A first alternate construction of the personal dispenser 20 is depicted in Figure 12, wherein like numerals are used to identify like components. The construction depicted in Figure 12 is generally identical to that shown in Figure 5, with the exception that the receptacle 38 incorporates an internal chamber 68 disposed within the internal cavity 58. The internal chamber 68 is defined by a generally conical barrier wall 70 which surrounds the outer circumference of the diaphragm pump 60, and extends upwardly therefrom. The generally conical barrier wall 70 has a cylindrical upper portion 72 with an upper opening 74. The diaphragm pump 60 corresponds identically to the diaphragm pump 60 shown in Figure 5 and described above, with the exception that the diaphragm pump 60 is completely or partially transparent, to permit visual inspection of the contents of the internal chamber 68.

While the personal dispenser 20 is coupled to the dispenser outlet 18, the generally conical barrier wall 70 prevents liquid received by the inlet port 40 from entering the internal chamber 68 until the level of the liquid reaches the height of the upper opening 74. Preferably, the height of the upper opening 74 is selected such that liquid will only enter the internal chamber 68 once the internal cavity 58 is almost completely full. This allows the presence or absence of liquid within the internal chamber 68 to serve as an indicator of whether or not the receptacle 38 is full. The presence or absence of liquid within the internal chamber 68 can furthermore be easily determined through visual inspection of the transparent diaphragm pump 60. In some embodiments, the liquid is coloured to be more readily visible through the transparent diaphragm pump 60.

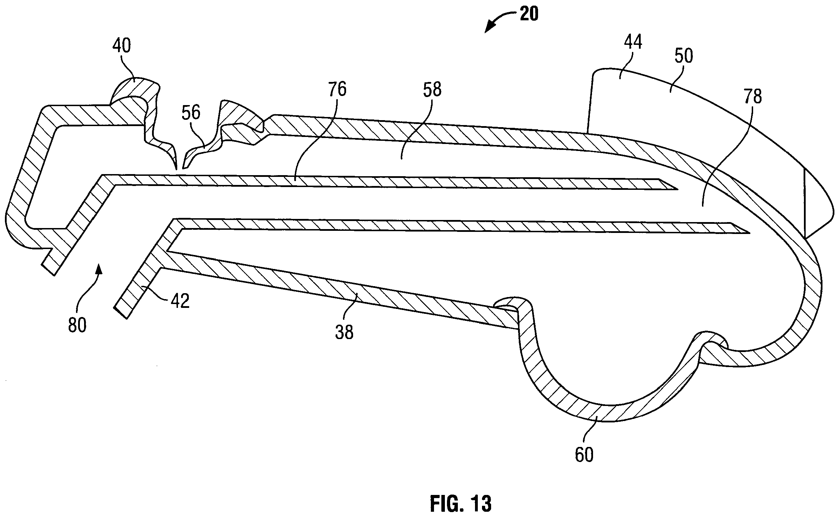

A second alternate construction of the personal dispenser 20 is depicted in Figure 13, wherein like numerals are used to identify like components. The construction depicted in Figure 13 is generally identical to that shown in Figure 5, with the exception that the baffle 62 has been omitted, and the outlet port 42 is formed as an elongated tubular member 76. The tubular member 76 extends from an inlet opening 78 disposed proximate to the generally arcuate docking seat 44 within the internal cavity 58, to a discharge opening 80 that protrudes out of the receptacle 38. Similar to the baffle 62 in the constructions described above, the height of the inlet opening 78 of the tubular member 76 prevents liquid received by the inlet port 40 from entering the outlet port 42 until the level of the liquid has reached the height of the inlet opening 78. This ensures that most of the air contained within the internal cavity 58 is expelled from the outlet port 42 before any liquid is discharged therefrom, in much the same way as the baffle 62 in the constructions described above.

Furthermore, when the personal dispenser 20 is removed from the master dispenser 10 and held manually upright or stood upright as shown in Figure 2B, the orientation of the tubular member 76 causes the inlet opening 78 to sit near the bottom of the personal dispenser 20. This allows almost all of the liquid contained in the receptacle 38 to be selectively discharged while in the upright position. In particular, the orientation of the tubular member 76 ensures that the inlet opening 78 remains in fluid communication with the liquid pooled at the bottom of the receptacle 38, even when the receptacle 38 is nearly empty.

The reservoir 12 may be replaced once the liquid contained therein has been depleted. When the reservoir 12 is empty, the personal dispenser 20 may advantageously be used as a backup supply of the liquid. The presence of this backup supply permits and may encourage users to wait until the reservoir 12 is completely empty before replacing the reservoir 12, and thus reduce the amount of liquid that is wasted when a replaceable reservoir 12 is replaced before it is completely empty.

Referring again to Figures 1 and 2C, both of these figures show the same condition in which the personal dispenser 20 is coupled to the master dispenser 10 and the piston forming element 402 is in an uppermost position under the bias of the spring 126 as permitted by the actuator 22. In this coupled position as seen in Figure 1, the personal dispenser 20 is below the shroud 19 with a lower profile of the shroud 19 and an upper profile of the personal dispenser 20 matching each other so as to provide a pleasing visual appearance. From the position of Figures 1 and 2C, on movement of the front distal end of the manual actuator 22 downwardly, the piston forming element 402 is moved downwardly relative to the piston chamber forming body 401. Since the piston chamber forming body 401 is fixed to the housing 14, the piston forming element 402 together with the discharge outlet 18 and the personal dispenser 20 are moved vertically relative to the shroud 19 and the housing 14 with operation of the pump assembly 16.

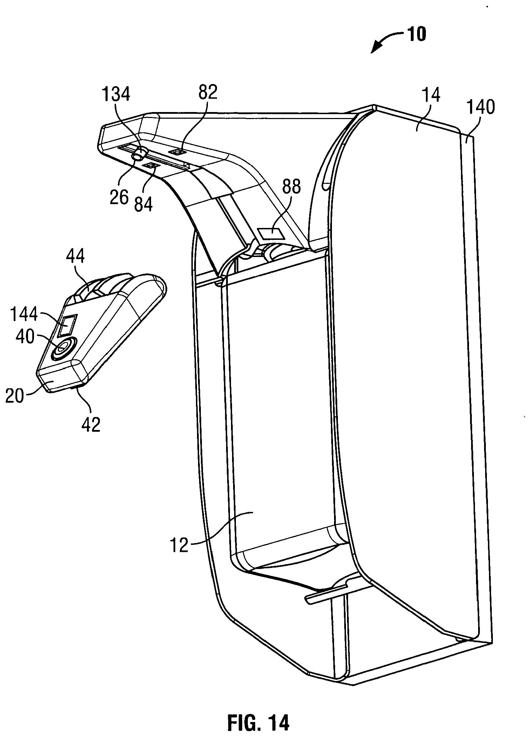

Figure 14 shows a liquid master dispenser 10 in accordance with a second preferred embodiment of the invention, wherein like reference numerals are used to denote like components. The liquid master dispenser 10 according to the second preferred embodiment is identical to the liquid master dispenser 10 according to the first preferred embodiment, with the exception that the manual actuator 22 has been omitted, and the master dispenser 10 has been adapted to incorporate electronic components to permit automatic and touchless activation of the pump assembly 16. The master dispenser 10 may, for example, incorporate features similar to those described in United States Patent No. 5,836,482 to Ophardt et al., issued November 17, 1998, and United States Publication No. US 20090045221 to Ophardt et al., published February 19, 2009.

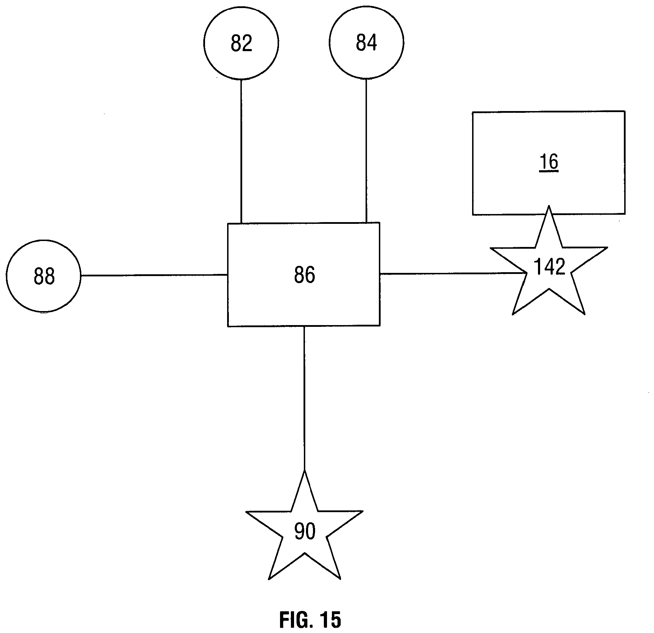

The master dispenser 10 incorporates a back panel 140 which is coupled to the housing 14 and carries various additional components for touchless operation of the master dispenser 10 including the provision as schematically shown on Figure 15 of an electric motor 142 which when activated moves the piston element 402 in a cycle of operation to dispense an allotment of fluid from the pump assembly 16, various sensors 82, 84 and 88, a central processing unit 86, and a communication system 90. The central processing unit 86 is connected to various electronic components, as shown schematically in Figure 15. The central processing unit 86 acts as a control system for the pump assembly 16, and is able to activate the pump assembly 16 using the electric motor 142.

The housing 14 of the master dispenser 10 is modified to incorporate infrared sensors 82 and 84, which are positioned adjacent to the outlet end 26 of the spout tube 134. The personal dispenser sensor 82 is configured to detect if the personal dispenser 20 is coupled to the spout tube 134. The sensor 82 comprises an emitter of infrared radiation and a sensor of infrared radiation. The sensor 82 is able to detect changes in infrared radiation emitted that is received by its sensor, and is calibrated to recognize the infrared radiation that is sensed due to reflection from the personal dispenser 20 when the personal dispenser 20 is placed immediately adjacent to the sensor 82, as occurs when the personal dispenser 20 is coupled to the spout tube 134.

The fluid level sensor 84 is configured to detect if the receptacle 38 of the personal dispenser 20 contains a preselected volume of the liquid. The sensor 84 comprises an emitter of infrared radiation and a sensor of infrared radiation. To enable the operation of the sensor 84, the personal dispenser 20 is provided with a transparent window 144 which permits infrared radiation to pass therethrough. The window 144 is positioned to align with the sensor 84 when the personal dispenser 20 is coupled to the spout tube 134 so that infrared radiation from the emitter of the sensor 84 passes through the window 114 into the receptacle 38, and the sensor of the sensor 84 receives infrared radiation reflected from within the receptacle 38 back out through the window 144. The sensor 84 is able to detect changes in the infrared radiation reflected back through the window 144 from within the receptacle 38. The sensor 84 is calibrated to recognize the change in infrared radiation that occurs when the liquid within the receptacle 38 rises to one or more heights including preferably a height corresponding to the preselected volume.

An additional infrared hand sensor 88 is also incorporated into the housing 14 near the spout tube 134. The sensor 88 is configured to detect the presence of a user's hand immediately below the outlet end 26 when the personal dispenser 20 is removed, and below the outlet port 42 when the personal dispenser 20 is coupled to the spout tube 134. The sensor 88 comprises an emitter of infrared radiation and a sensor of infrared radiation. The sensor 88 is calibrated to recognize the change in infrared radiation that occurs when a person's hand is held in the area below the spout tube 134. The sensors 82, 84 and 88 are linked to the central processing unit 86 by being wired or wireless connections as schematically shown on Figure 15.

During operation, the master dispenser 10 is configured to automatically fill or refill the personal dispenser 20. In particular, the sensor 82 is configured to detect the coupling of the personal dispenser 20 to the spout tube 134, and to communicate this information to the central processing unit 86. This triggers the central processing unit 86 to activate the pump assembly 16 via the electric motor 142, to pump the liquid from the reservoir 12 out through the spout tube 134 and into the receptacle 38 of the personal dispenser 20. The pump assembly 16 remains activated until the sensor 84 detects that the receptacle 38 is full to the preselected volume, and transmits this information to the central processing unit 86. The central processing unit 86 then deactivates the pump assembly 16.

After the personal dispenser 20 has been filled and while the personal dispenser 20 is coupled to the spout tube 134, the pump assembly 16 can be activated to discharge the liquid from the master dispenser 10. In particular, the central processing unit 86 is configured to activate the pump assembly 16 when the sensor 88 detects the presence of a user's hand underneath the spout tube 134. This occurs regardless of whether or not the personal dispenser 20 is coupled to the spout tube 134, and regardless of the volume of liquid contained within the personal dispenser 20.

In a preferred embodiment of the invention, the master dispenser 10 is configured for monitoring hand cleaning compliance by automatically recording the amount of liquid that is discharged from the spout tube 134. Since the master dispenser 10 is constructed such that both (i) the liquid that is used to fill the personal dispenser 20, and (ii) the liquid that is directly dispensed, as for example onto a user's hand, are discharged from the spout tube 134, both uses of the liquid (i) and (ii) are automatically taken into account. This may be useful, for example, in a hospital setting where both personal dispensers 20 and stationary master dispensers 10 are frequently used, and the use of which must both be monitored in order to accurately assess hand cleaning frequency.

Information indicative of the amount of liquid dispensed may be collected, for example, by recording each activation of the pump assembly 16; by monitoring the volume of the liquid within the reservoir 12; and/or by detecting the flow of liquid through the pump assembly 16 or the spout tube 134. This information is collected by the central processing unit 86, which may transmit the information via the communication system 90 to a central computer, where it can be compiled and made available to hospital staff. The monitoring system may, for example, operate in a similar manner to that described in the applicant's co-pending Canadian Patent Application No. 2865608.

In a dispenser as shown in Figure 14, the personal dispenser 20 is advantageously able to act as a manual backup pump in the event that the master dispenser 10 loses power, as explained below with reference to Figure 2C, assuming that the lever 22 is to be moved by the electric motor 142 not shown in Figure 2C. If power is lost, with the result that the electric motor 142 is inoperable, while the personal dispenser 20 is coupled to the master dispenser 10 as seen in Figure 2C, the internal cavity 58 of the receptacle 38 can be manually pressurized through compression of the diaphragm pump 60, to thereby discharge the liquid contained in the receptacle 38 out the outlet port 42 onto the user's hand 17. The diaphragm pump 60 is biased toward returning to its outwardly bulging semi-spherical shape upon release of the manual compressing force so as to increase the volume of the internal cavity 58 and produce a resulting drop in pressure or vacuum within the internal cavity 58 of the receptacle 38 sufficiently great to draw the liquid contained in the reservoir 12 into the receptacle 38. That is, the vacuum pressure within the internal cavity 58 of the receptacle 38 is sufficiently great to draw liquid through the one-way inlet valve 56 and through the pump assembly 16. In this regard, the vacuum is sufficient to draw fluid in the reservoir 12 up through the dip tube 128, through the one way valve 132, the piston chamber 124, the one-way valve 130, the internal conduit 120 and the one-way inlet valve 56, and into the internal cavity 58 of the receptacle 38. In this way, the liquid may be manually drawn from the reservoir 12 and out through the outlet port 42 in individual allotments through repeated manual compression and release of the diaphragm pump 60.

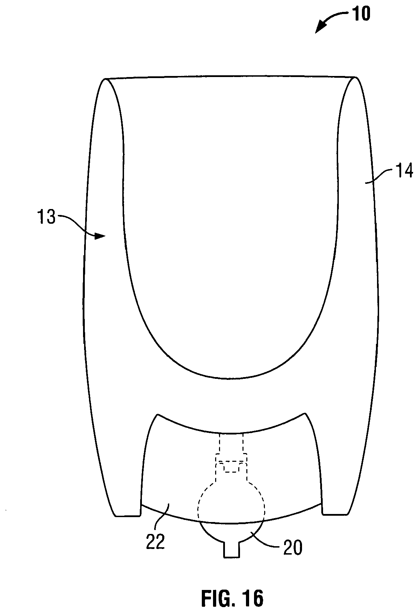

Reference is made to Figures 16 to 22 which show a liquid master dispenser 10 in accordance with a third preferred embodiment of the invention, wherein like reference numerals are used to denote like components. As in the embodiments described above, the master dispenser 10 includes a reservoir 12, a housing 14, a pump assembly 16, a dispenser outlet 18, and a personal dispenser 20. The master dispenser 10 may have a similar construction to that disclosed in United States Patent No. 7,984,825 to Ophardt et al., issued July 26, 2011; United States Patent No. 8,684,236 to Ophardt, issued April 1, 2014; and/or the applicant's co-pending Canadian Patent Application No. 2839615.

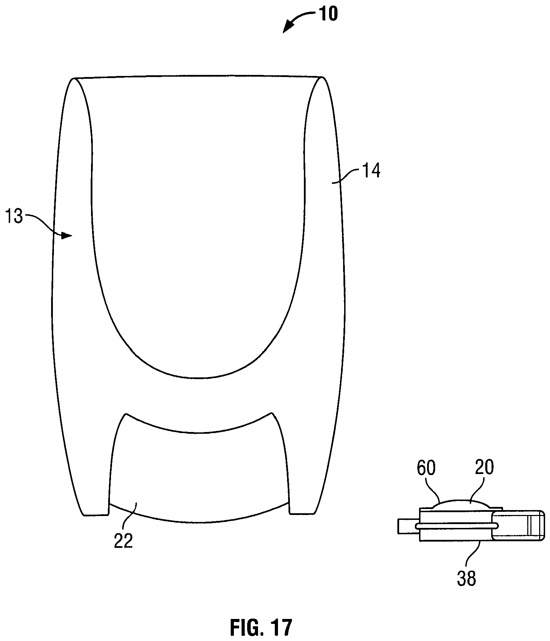

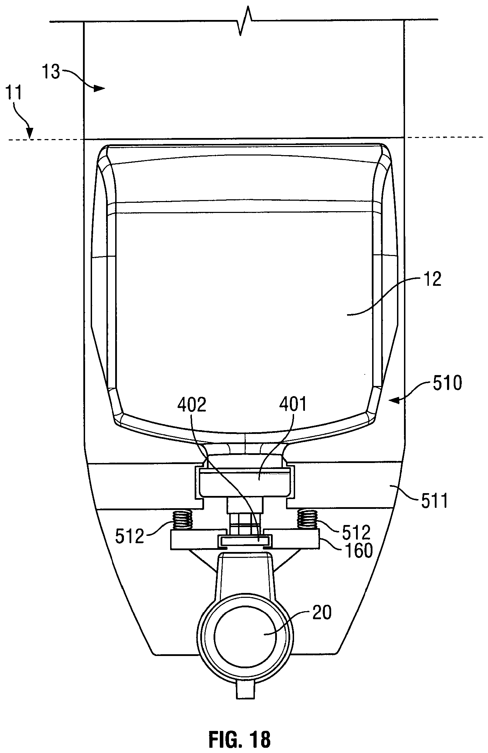

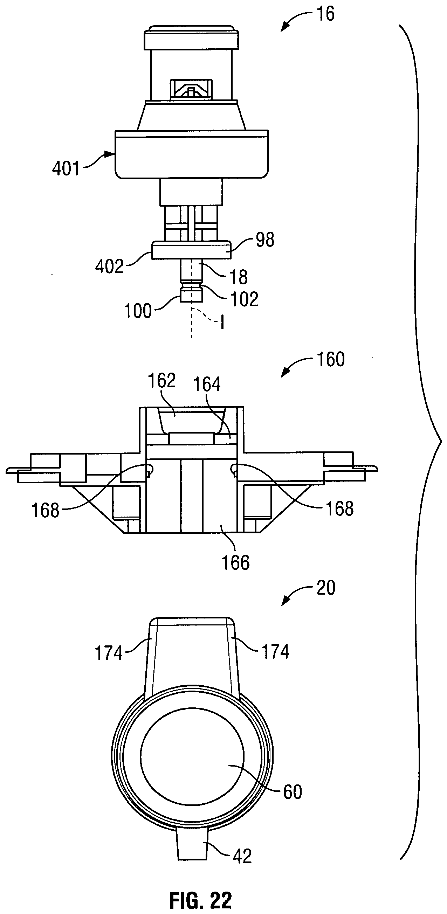

Figure 16 shows a front view in which a front cover 13 can be seen as well as a manual actuator 22, with lowermost portions of a personal dispenser 20 extending downwardly below the manual actuator 22. The front cover 13 is coupled to the housing 14 shown in Figure 16 in a conventional manner as by being hingedly coupled thereto for movement about a hinge axis 11 from a closed position as seen in Figure 16 enclosing the reservoir 12 and an open position shown in Figure 18 in which the housing 14 and reservoir 12 are accessible. The housing 14 is schematically shown in Figure 18 as having a back plate 510 adapted for attachment to a wall. A support plate 511 is fixed to the back plate 510 and extends forwardly to removably support the piston chamber forming body 401 of the pump assembly 16 and thereby the reservoir 12 and the piston forming element 402. An actuator plate 160 is schematically shown as mounted to the housing 14 for reciprocal upward and downward sliding movement relative the support plate 511. Spring members 512 are shown disposed between the support plate 511 and the actuator plate 160 to bias the actuator plate 160 downwardly away from the support plate 511. The actuator plate 160 removably engages the piston forming element 402. The front cover 13 carries a manual actuator 22 shown only in Figures 16 and 17 which, when the front cover 13 is in the closed position, is mechanically coupled between the housing 14 and the actuator plate 160 in such a way that manual movement of the manual actuator 22 causes the actuator plate 160 to slide upwards relative the support plate 511 against the bias of the spring members 512. The actuator plate 160 is best shown in Figure 22 as having an upper chamber 162 with an actuation flange 164 for engagement with the piston forming element 402 of pump assembly 16, and a lower chamber 166 with a pair of small lateral protrusions 168 for engagement with the personal dispenser 20.

The reservoir 12, the pump assembly 16, the dispenser outlet 18, and the personal dispenser 20 are coupled to form a replaceable cartridge 94, which is shown in Figure 18. The reservoir 12 is a container for holding the liquid to be dispensed from the liquid dispenser 10. The liquid is preferably hand soap or hand sanitizer. The bottom of the reservoir 12 is provided with a reservoir outlet 96, which is coupled to the piston chamber forming body 401 of pump assembly 16.

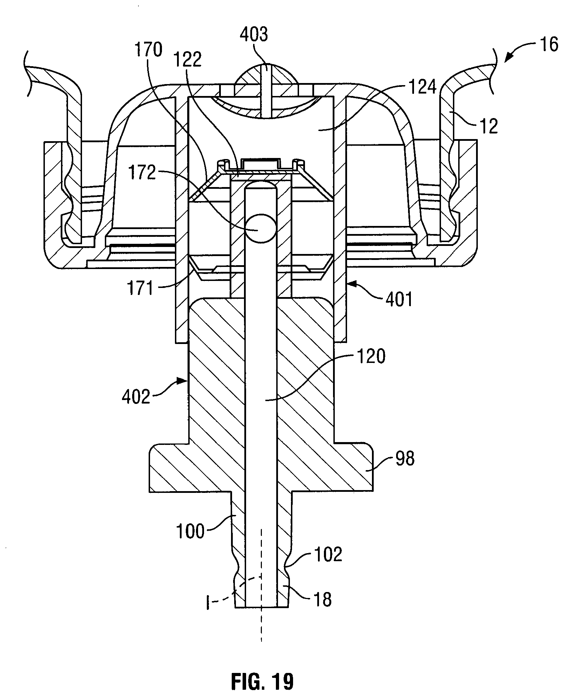

The pump assembly 16 is shown in Figure 19 with the piston chamber forming body 401 threadably secured to the outlet 96 of the reservoir 12. The piston forming element 402 of the pump assembly 16 includes an actuation collar 98, configured to removably be received within the upper chamber 162 of the actuator plate 160 to couple the piston forming element 402 and the actuator plate 160, such that with reciprocal upwards and downwards movement of the actuator plate 160 the piston forming element 402 is reciprocally moved coaxially relative the piston chamber forming body 401 to pump the liquid from the reservoir 12 out through the dispenser outlet 18. Thus, by manual movement of the manual actuator 22, an upwards mechanical force is applied to the actuation collar 98 and transferred to the piston forming element 402 to pump the liquid from the reservoir 12.

The internal structure of the pump assembly 16 is best shown in Figure 19. As in the previously described embodiments, the pump assembly 16 includes a piston forming element 402 with a piston 122 that is positioned within a piston chamber 124 defined within the piston chamber forming body 401. The dispenser outlet 18 is in fluid communication with the piston 122 via an internal conduit 120 with a one-way valve 403 across the conduit permitting flow outwardly from the reservoir 12 but preventing flow inwardly into the reservoir 12. The piston 122 is provided with an inner disc 170 which acts as a one-way valve to permit pressurized liquid between the inlet valve 403 and the inner disc 170 to flow outwardly past inner disc 170 in the piston chamber 124, and prevents liquid from flowing inwardly therepast. The piston 122 is provided with a sealing disc 170 which prevents liquid from flowing outwardly therepast in the piston chamber 124. With upward movement of the piston 122 relative the piston chamber forming body 401 in a retraction stroke, the liquid within the piston chamber 124 between the inlet valve 403 and the inner disc 170 is forced past the inner disc 170 and through an opening 172 into the internal conduit 120 of the hollow stem of the piston forming element 402 and out the dispenser outlet 18. With downward movement of the piston 122 relative the piston chamber forming body 401 in a withdrawal stroke, the volume between the inlet valve 403 and the inner disc 170 increases drawing liquid from the reservoir 12 outwardly past the inlet valve 403.

The dispenser outlet 18 is formed as a short outlet tube 100 that extends from the pump assembly 16 along an insertion axis I. A coaxial annular groove 102 is formed on the external surface of the outlet tube 100.

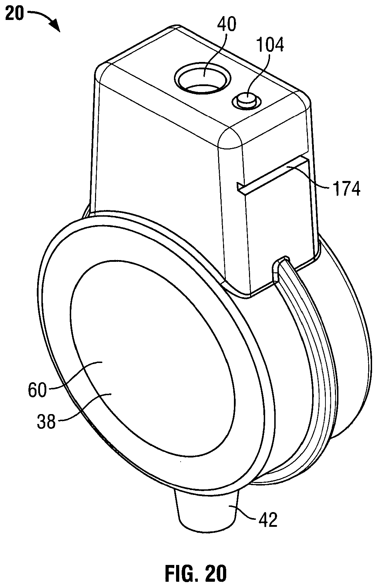

The personal dispenser 20 is best shown in Figure 20. As in the embodiments described above, the personal dispenser 20 includes a receptacle 38, an inlet port 40, and an outlet port 42. In addition, the personal dispenser 20 incorporates a pressure relief valve 104 and lateral channels 174. As in the previous embodiments, the inlet port 40 is configured to receive the liquid discharged from the dispenser outlet 18; the receptacle 38 is configured to contain the liquid received by the inlet port 40; and the outlet port 42 is configured to discharge the liquid from the receptacle 38.

As is best shown in Figure 17, the receptacle 38 incorporates an outwardly bulging diaphragm pump 60. As in the previous embodiments, the diaphragm pump 60 is manually compressible to expel the liquid contained in the receptacle 38 and biased to return to an initial unbiased position.

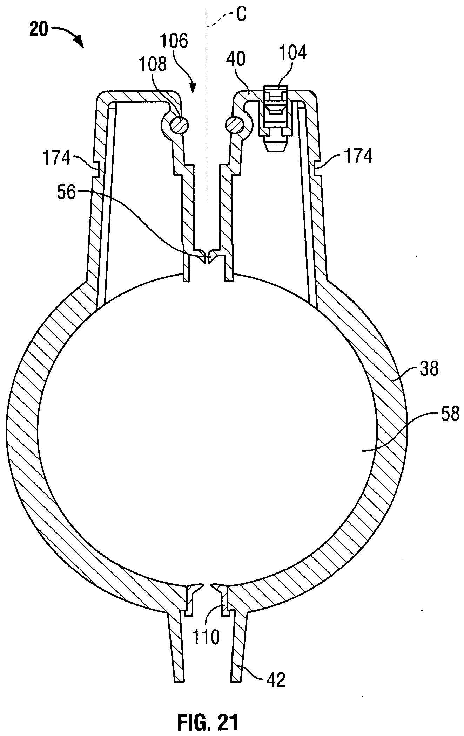

The internal structure of the personal dispenser 20 is best shown in Figure 21. The inlet port 40 defines a coupling channel 106 that extends along a coupling axis C. The coupling channel 106 is configured to receive the outlet tube 100 therein to couple the personal dispenser 20 and the dispenser outlet 18. An O-ring 108 is disposed coaxially within the coupling channel 106, and is arranged to engage with the annular groove 102 of the outlet tube 100 in a snap fit when the outlet tube 100 is received within the coupling channel 106. The engagement of the O-ring 108 with the annular groove 102 provides a preselected degree of resistance against the removal of the outlet tube 100 from the coupling channel 106. The degree of resistance is selected to prevent unintentional uncoupling of the personal dispenser 20 from the dispenser outlet 18, while permitting the personal dispenser 20 to be coupled and uncoupled when so desired.

The external laterally extending lateral channels 174 on personal dispenser 20 are configured to engage in a snap fit with the lateral protrusions 168 on the actuator plate 160, to provide further resistance against unintentional uncoupling of the personal dispenser 20. In particular, the engagement of the lateral channels 174 with the lateral protrusions 168 ensures that the personal dispenser 20 moves up and down with the actuator plate 160 when the manual actuator 22 is pressed, so as to remain coupled to the dispenser outlet 18.

The receptacle 38 is preferably formed from molded plastic, and defines an internal cavity 58 within which the liquid received from the inlet port 40 accumulates. The outlet port 42 is situated at the bottom of the receptacle 38, and includes a one-way outlet valve 110 configured to discharge fluid from the receptacle 38 only when a pressure differential across the outlet port 42 exceeds a preselected threshold.

To couple the personal dispenser 20 to the dispenser outlet 18, the outlet tube 100 is aligned with the coupling channel 106, such that the insertion axis I and the coupling axis C are coaxial, and the outlet tube 100 is inserted into the coupling channel 106. With the personal dispenser 20 coupled to the dispenser outlet 18, by manually pressing the manual actuator 22, the pump assembly 16 is activated to dispense the liquid from the reservoir 12 out through the outlet tube 100 and into the inlet port 40. The liquid then flows down into the internal cavity 58 of the receptacle 38, and begins accumulating therein.

As the liquid accumulates in the receptacle 38, the air displaced thereby is expelled through the pressure relief valve 104. This prevents the pressure within the internal cavity 58 from increasing to above the threshold pressure for the outlet valve 110, and so the outlet valve 110 does not open. Once the level of the liquid within the receptacle 38 reaches the height of the pressure relief valve 104, the pressure relief valve 104 closes and the pressure within the receptacle 38 may then rise to above the threshold pressure for the outlet valve 110 with liquid to be discharged out the outlet valve 110. The function of the pressure relief valve 104 may be achieved, for example, using a float that raises with the liquid to seal the pressure relief valve 104 when the level of the liquid within the receptacle 38 reaches the height of the pressure relief valve 104.

The personal dispenser 20 may be uncoupled from the dispenser outlet 18 once the desired volume of liquid has accumulated therein for remote use, or the personal dispenser 20 may be left coupled to the dispenser outlet 18. As in the previous embodiments, the construction of the liquid dispenser 10 advantageously permits the liquid to be discharged while the personal dispenser 20 remains coupled to the dispenser outlet 18. In particular, once the level of the liquid within the internal cavity 58 has reached the height of the pressure relief valve 104, the internal cavity 58 may be pressurized to create a pressure differential across the outlet port 42 that exceeds the preselected threshold, and thereby open the discharge valve 110 to dispense the liquid. As in the previous embodiments, the internal cavity 58 may be pressurized by introducing further liquid into the receptacle 38 through activation of the pump assembly 16, or by manual compression of the diaphragm pump 60.

The personal dispenser 20 can be easily uncoupled from the dispenser outlet 18 when desired, to provide a mobile supply of the liquid. As in the previous embodiments, the liquid is discharged from the personal dispenser 20 by manually compressing the diaphragm pump 60. Preferably, the pressure relief valve 104 is configured to remain closed while the personal dispenser 20 is uncoupled from the dispenser outlet 18.

As in the previous embodiments, the pump assembly 16 remains operable to dispense the liquid directly from the dispenser outlet 18 as onto a user's hand when the personal dispenser 20 is removed from the dispenser outlet 18.

When the supply of liquid within the reservoir 12 is depleted, the front cover 13 of the housing 14 can be hinged open to provide access to the replaceable cartridge 94 therein. The replaceable cartridge 94 can then easily be removed from the housing 14, and replaced with a new replaceable cartridge 94.

The invention is not limited to the preferred embodiments described herein. A person skilled in the art will appreciate that there are many possible variations and modifications that fall within the scope of the invention.

For instance, while the invention has been described in the preferred embodiments as for dispensing hand cleaners, the invention could also be applied to devices for dispensing other types of liquids such as hand cream, sunscreen, beverages, and other liquified cleaners, soaps and waxes. For example, in one alternate embodiment, it is envisioned that the invention could be applied to a device for dispensing car wax and/or polish in an auto body shop, to provide workers with portable refillable dispensers for small touch-ups.

While certain specific mechanisms for coupling and uncoupling the personal dispenser 20 and the dispenser outlet 18 have been described, it is to be appreciated that there may be many other suitable coupling mechanisms such as magnetic attachments, hook and loop fasteners, or elastic clips that fall within the scope of the invention. Furthermore, the coupling mechanisms that have been described with respect to one or more of the embodiments could optionally be used with the other embodiments, if desired. For example, the generally arcuate track 30 described with reference to the first embodiment shown in Figures 1 to 13 could, optionally, be used in a master dispenser 10 similar to the third embodiment shown in Figures 16 to 22. In particular, the actuator plate 160 could incorporate a generally arcuate track 30 for securely mounting the personal dispenser 20, and the personal dispenser 20 could be provided with a corresponding generally arcuate docking seat 44.

It is to be appreciated that the coupling mechanism could be configured to attach the personal dispenser 20 to any suitable part of the master dispenser 10 including, for example, the reservoir 12, the housing 14, the dispenser outlet 18, or the pump assembly 16, so long as the personal dispenser 20 is functionally linked to the master dispenser 10 in a way that allows liquid discharged from the master dispenser 10 to be received by the personal dispenser 20.

While the personal dispenser 20 has been described in some embodiments as optionally incorporating a transparent or semi-transparent diaphragm pump 60 for the purpose of visualizing the level of liquid therein, there may be many other suitable ways to determine the level of the liquid. For example, the personal dispenser 20 may incorporate a coloured float that rises with the level of the liquid. Alternatively, a transparent panel or chamber could be provided on the personal dispenser 20 through which the liquid is visible once it reaches a certain level.

While the preferred embodiments have described the receptacle 38 as incorporating a diaphragm pump 60 for the purpose of selectively discharging the liquid from the receptacle 38, it is to be understood that there are many alternative mechanisms that could be used. For example, the entire receptacle 38 could be formed from a flexible material such that the liquid is dischargeable by squeezing any part of the receptacle 38. The personal dispenser 20 could also incorporate other pump structures, such as an electric pump. Preferably, the personal dispenser 20 is configured to discharge between 1 ml and 5 ml of the liquid with each compression of the diaphragm pump 60, although any other desired discharge volume could be selected.

The liquid dispenser 10 may incorporate any suitable device for instructing the central processing unit 86 to activate the pump assembly 16, including but not limited to a button, a touchscreen, a wireless communication device, a microphone, a motion sensor, a radar sensor, a light sensor, an infrared sensor, or a camera.

In the first embodiment of Figures 1 to 13, three different configurations for the personal dispenser 20 are shown in which, while the personal dispenser 20 is coupled to the dispenser outlet 18 and full, operation of the master dispenser 10 discharges liquid through the personal dispenser 20 and onto a user's hand. This is preferred but not necessary. For example, each personal dispenser 20 could be configured such that while coupled to the dispenser outlet 18, once the personal dispenser 20 is filled with liquid, further operation of the pump assembly 16 will not discharge liquid out the personal dispenser 20. For example, the further operation of the pump assembly 16 after the personal dispenser 20 is full will not discharge any further liquid. The personal dispenser 20 must be removed from the dispenser outlet 18 for liquid to be discharged onto a user's hand from the dispenser outlet 18, or the personal dispenser 20 manually operated to discharge fluid from its outlet port 42 while the personal dispenser 20 is coupled to the dispenser outlet 18. In any case, preferably an arrangement is provided for detecting the amount of liquid discharged from the master dispenser 10 whether directly onto a user's hand or into a personal dispenser 20.

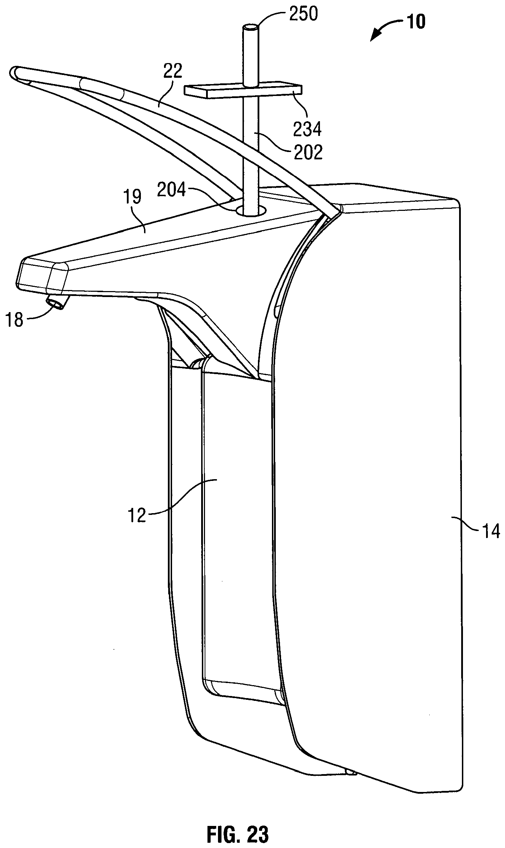

Reference is made to Figures 23 to 26 which show a liquid master dispenser 10 in accordance with a fourth preferred embodiment of the invention. The fourth embodiment of Figures 23 to 26 is identical to the first embodiment of Figures 1 to 11, with the exceptions that (a) a two way valve mechanism 200 including a refill tube 202 are provided on the spout tube 134, and (b) an opening 204 is provided through the shroud 19.

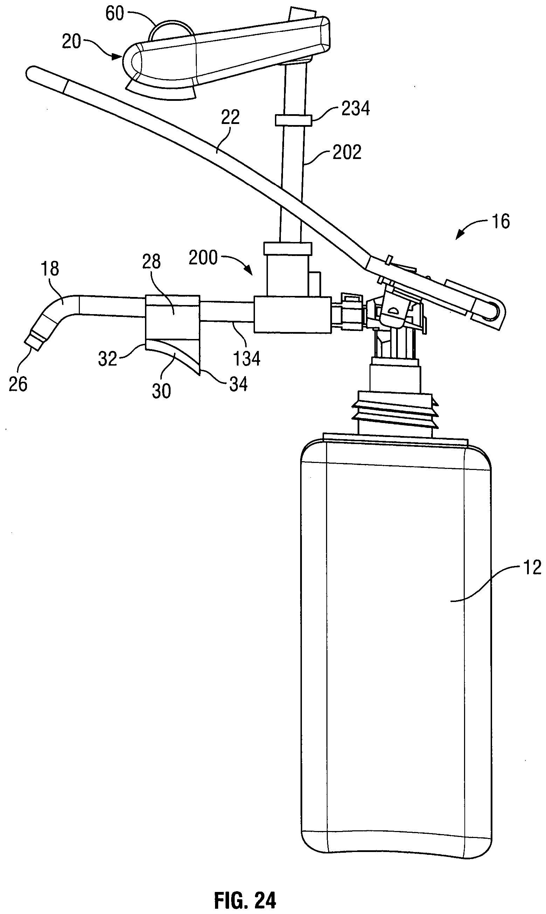

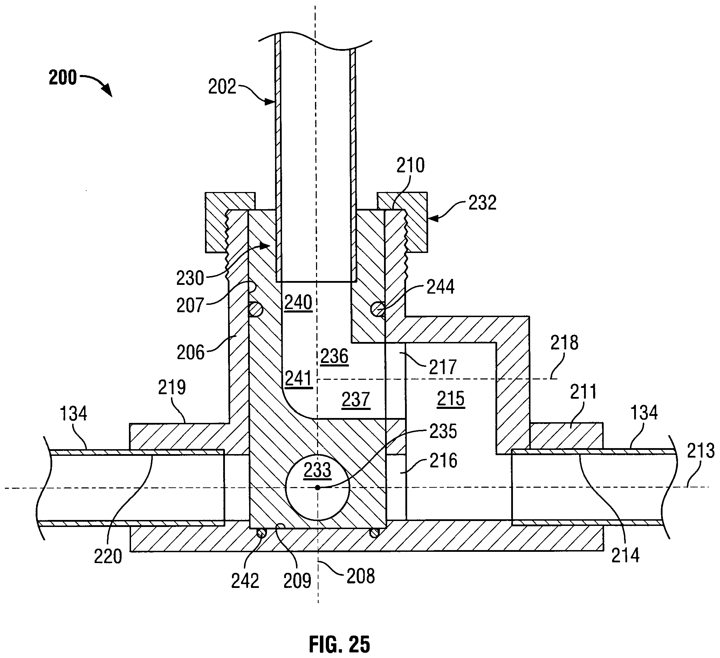

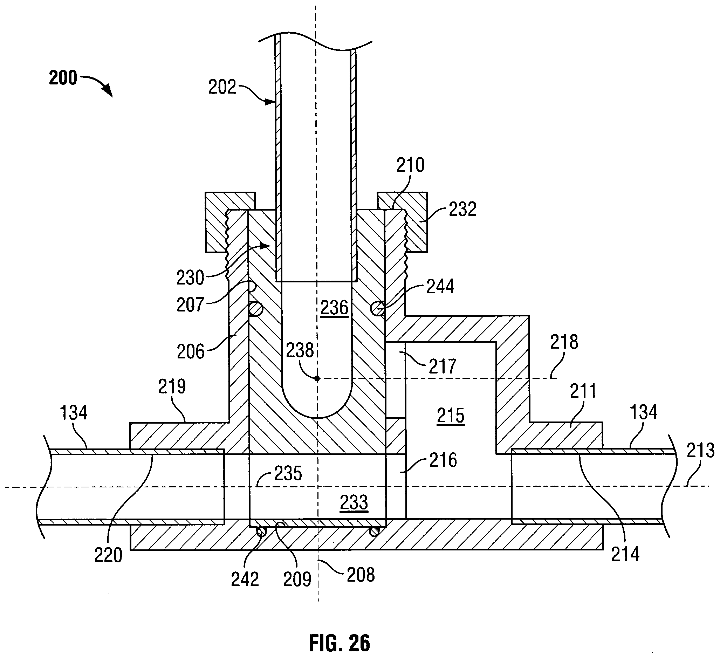

The valve mechanism 200 is movable between a flow through condition as in Figures 23 and 26 in which fluid from the pump assembly 16 is directed merely to the discharge outlet 18, and a diversion condition as in Figures 24 and 25 in which fluid from the pump assembly 16 is directed merely to the refill tube 202.

Reference is made to Figure 25 which shows a vertical cross-section through the valve mechanism 200. The valve mechanism 200 includes a valve housing 206, a valve member 230 and a cap 232.

The housing 206 defines a cylindrical bore 207 disposed about a vertical axis 208. The bore 207 is closed at a lower end 209 and open at an upper end 210.

The valve housing 206 has a cylindrical inlet collar 211 disposed about a lower horizontal axis 213. The inlet collar 211 receives coaxially therein an inlet portion 214 of the spout tube 134 which receives fluid from the pump assembly 16. The inlet collar 211 opens into a diverter chamber 215 which has two cylindrical outlet ports. A lower outlet port 216 opens into the bore 207 and is coaxial about the lower horizontal axis 213. An upper outlet port 217 opens into the bore 207 and is coaxial about an upper horizontal axis 218 spaced above the lower horizontal axis 213. The housing 206 has a cylindrical lower outlet collar 219 which opens into the bore 207 diametrically across from the lower outlet port 216 and is coaxial about the lower horizontal axis 213. The lower outlet collar 219 securely receives therein an outer portion 220 of the spout tube 134 which extends to the discharge outlet 18.

The valve member 230 is cylindrical and sized to be coaxially received within the bore 207 and for relative rotation therein about the vertical axis 208 between a flow-through condition as seen in Figure 26 and a diversion condition as seen in Figure 25 by rotation of the valve member 230 90 degrees about the axis 208, the refill tube 202 is fixedly secured to the valve member 233 with the refill tube 202 coaxial about the vertical axis 208. As can be seen in Figure 23, a handle member 234 is fixedly secured to the refill tube 202 for rotation therewith and for movement between a position corresponding to the flow-through condition as shown in Figure 23 in which the handle extends forwardly and rearwardly and a position corresponding to the diversion condition as shown in Figure 24 in which the handle extends side to side.

The valve member 230 has two flow passageways therethrough. A cylindrical lower passageway 233 extends through the valve member 230 along a horizontal axis 235. An L-shaped diverter passageway 236 extends through the valve member 230 with a horizontal portion 237 about a horizontal axis 238 and a vertical portion 240 coaxial about the axis 208, and an arcuate portion 241 which joins the horizontal portion 237 and the vertical portion 240.

The refill tube 202 is fixedly coaxially received within an upper end of the vertical portion 240.

As seen in Figure 25, corresponding to the diversion condition, the diverter passageway 236 is open to the diverter chamber 215 via the upper outlet port 217 such that fluid flow from the inlet portion 214 of the spout tube 134 passes through the diverter chamber 215 into the diverter passageway 236 and to the refill tube 202. In the position as seen in Figure 25, the through passageway 233 is disposed to extend perpendicular to the lower horizontal axis 218 and thus fluid flow through the bore 207 from the diverter chamber 25 to the lower outlet collar 219 is prevented.

Referring to Figure 26, which represents the flow-through condition, the flow- through passageway 233 is disposed coaxially with the lower horizontal axis 213 and fluid from the pump assembly 16 may flow from the inlet portion 214 of the spout tube 134 through the diverter chamber 215 through the lower outlet port 216 into the bore 207 through the flow-through passageway 233 in the valve member 230 and out the outlet collar 219 to the outer portion 220 of the spout tube 134 and hence to the discharge outlet 18. Flow from the diverter chamber 215 to the refill tube 202 is blocked since the valve member 230 blocks the upper outlet port 217.

The valve member 230 is axially retained within the bore 207 by a threaded cap 232 which engages an axial shoulder on the valve member 230 urging a lower end of the valve member 230 downwardly onto a first sealing ring 242 disposed between the lower end of the valve member 230 and the lower end 209 of the bore 207 coaxially about the vertical axis 208. A second sealing ring 244 is coaxially about the vertical axis 208 providing a seal between a cylindrical inner surface of the bore 207 and a cylindrical outer surface of the valve member 230.

As can be seen in Figure 23, the refill tube 202 extends upwardly through the shroud 19 through the opening 204. The opening 204 permits the refill tube 202 to move vertically with the spout tube 134 relative the shroud 19 during an operation of the pump assembly 16 to dispense fluid.

The refill tube 202 has a refill outlet 250 preferably having a configuration similar to the outlet end 26 of the discharge outlet 18 such that a personal dispenser 20 of the type illustrated in Figure 5 may be coupled to the refill outlet 250 as illustrated in Figure 24. With the valve mechanism 200 in the diverted condition as seen in Figure 24 or Figure 26 with operation of the pump assembly 16, fluid is discharged into the personal dispenser 20 to fill the same by manual operation of the actuator 22. When the personal dispenser 20 becomes filled with liquid as may be seen, for example, by a person using the dispenser 10 seeing the fluid as filling the transparent diaphragm pump 60 in the personal dispenser 20, manual operation of the pump assembly 16 is ceased, the handle 234 may be rotated 90 degrees from the diversion condition to the flow-through condition as shown in Figure 23 and the personal dispenser 20 removed from the refill tube 202 for remote use.

Figure 24 shows a mounting dock 28 coupled to the spout tube 134. With a mounting dock 28 coupled to the spout tube 134, then a personal dispenser 20 may also be coupled to the discharge outlet 18 as with the first embodiment of Figure 1. However, in the fourth embodiment, the provision of a mounting dock 28 on the spout tube 134 is not necessary.

In the fourth embodiment shown in Figure 24, a personal dispenser 20 is shown as coupled to the refill tube 202 with the personal dispenser 20 having the same configuration as in Figure 5. This, of course, is not necessary and a personal dispenser with a different configuration may be provided adapted for removable coupling to the refill tube 202 for filling. For example, a different personal dispenser might be arranged so as to extend more vertically upwardly from the refill tube 202 effectively coaxially about the refill tube 202 as for balancing of the personal dispenser upon the refill tube 202. The personal dispenser may be configured such that when the personal dispenser is full of the liquid, the flow of fluid out of the outlet of the personal dispenser 20 is prevented.

In accordance with the fourth embodiment of the invention, the discharge outlet 28 may include a spray nozzle in its outlet 26 of a type, for example, to spray small droplets or a mist of the liquid being dispensed as can be accommodated by providing restrictions or rotating members with the outlet 26. Similarly, the personal dispenser 20 may have, in addition to a one-way outlet valve 66, a discharge outlet which also provides such a spray nozzle to spray small droplets or a mist of the liquid when the personal dispenser such is manually operated. In the context of the arrangement in Figure 24, insofar as the dispenser outlet 26 has a spray nozzle, it may be advantageous to refill the personal dispenser 20 from the refill nozzle rather than through the outlet 26.

The master dispenser 10 can incorporate additional mechanisms for discharging the liquid from the reservoir 12. For example, as in the fourth embodiment of Figures 23 to 26, liquid discharged by a single pump assembly 16 is delivered by suitable valving arrangements such as the two-way valve assembly 200 either to the dispenser outlet 18 or to a separate and distinct refill outlet 250 as on a refill tube 202 outlet, with a personal dispenser 20 to be removably attached to the refill outlet. The valving arrangements could have many alternate configurations to the two-way arrangement like that of the fourth embodiment which permit a manual selection as to which one of a number of outlets the liquid is to be dispensed, for example, through rotating or sliding a movable valve member to selectively choose which outlet receives fluid from an internal conduit 120 of the pump assembly 16. More than one such refill outlet may be provided. More than one discharge outlet may be provided.

A first alternate arrangement can be such that: (a) while a personal dispenser 20 is coupled to a refill outlet, fluid does not flow to the dispenser outlet 18 and fluid only flows out the refill outlet into the personal dispenser 20 until the personal dispenser 20 is full; (b) the liquid does not flow out of the personal dispenser 20 until the personal dispenser 20 is uncoupled from the refill outlet; (c) while the personal dispenser is not coupled to the refill outlet fluid merely flows to the discharge dispenser outlet 18 and onto a user's hand.

A second alternate arrangement can be such that: (a) while a personal dispenser 20 is coupled to the refill outlet, fluid does not flow to the dispenser outlet 18 and fluid only flows out the refill outlet into the personal dispenser 20 until the personal dispenser 20 is full; (b) the liquid does not flow out of the personal dispenser 20 until the personal dispenser 20 is uncoupled from the refill outlet; (c) while the personal dispenser 20 is coupled to the refill outlet and the personal dispenser 20 is full of liquid, liquid flows to the dispenser outlet 18 and onto a user's hand; and (d) while the personal dispenser 20 is not coupled to the refill outlet, fluid merely flows to the dispenser outlet 18 and onto a user's hand.

The master dispenser 10 could be provided with two different pump assemblies 16 with one to have liquid it discharges flow to the dispenser outlet 18 and onto a user's hand, and a second pump assembly to have the liquid it discharges flow to a refill outlet separate from the dispenser outlet 18, and adapted to have personal dispensers 20 removably coupled thereto for merely refilling. The pump assemblies 16 could be activated manually or driven by electric motors 142. Selection of the pump assembly 16 activated will determine where the liquid is delivered. Suitable selection of which pump assembly 16 may be activated can take into account whether or not a personal dispenser 20 is coupled to a refill outlet.

Any refill outlet which is separate from the dispenser outlet 18 may share some or all of their liquid pumping and delivery components with the dispenser outlet 18, or may be formed as entirely separate structures which do not share some or all of their liquid pumping and delivery components with the dispenser outlet 18. Preferably, all liquid dispensed is from the same reservoir 12. Preferably, however, an arrangement is provided for detecting the amount of liquid discharged from the master dispenser 10 whether directly onto a user's hand or into a personal dispenser 20.

Any suitable sensor arrangement for detecting the amount of liquid discharged from the master dispenser 10 could be used including, for example, ones that (i) detect a volume or a mass of the liquid contained in the reservoir 12; (ii) detect a flow of the liquid through the dispenser outlet 18; (iii) detect a flow of the liquid through the refill outlet; (iv) detect the activation of the discharge mechanism; or (v) detect the activation of the filling mechanism.

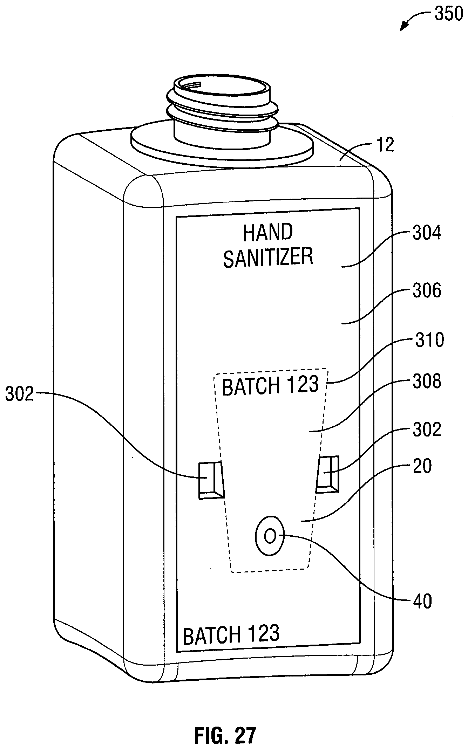

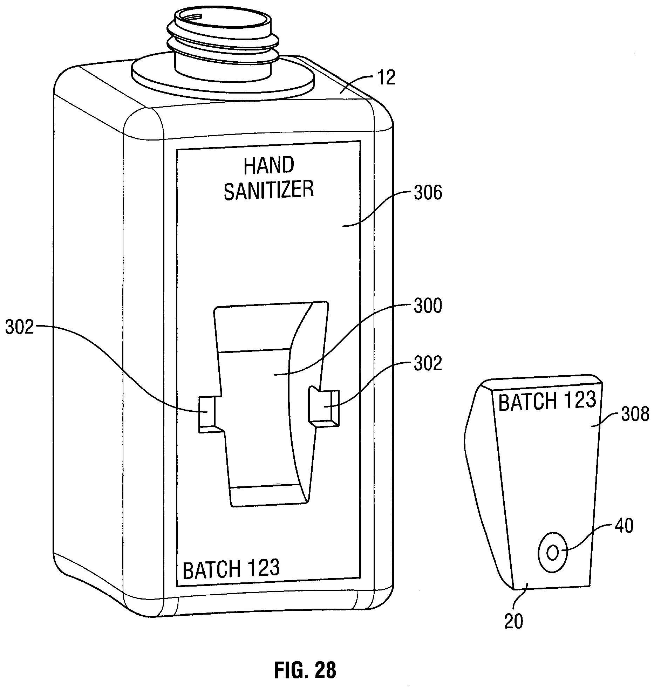

Figures 27 and 28 show a reservoir cartridge 350 in accordance with a fifth preferred embodiment of the invention, wherein like reference numerals are used to denote like components. The reservoir cartridge 350 comprises a reservoir 12 and a personal dispenser 20, which are configured to be used with a liquid master dispenser 10 in a similar manner as in the embodiments described above. What distinguishes the fifth embodiment of the invention from the previously described embodiments, is that the reservoir 12 and the personal dispenser 20 are packaged together in an initial bundled configuration for distribution. In particular, the reservoir 12 has a cavity 300 that is shaped to receive the personal dispenser 20 therein. The personal dispenser 20 is configured so as not to protrude from the cavity 300, with the face of the personal dispenser 20 approximately flush with the face of the reservoir 12. This gives the reservoir cartridge 350 a relatively flat and smooth outer profile, which permits the cartridge 350 to be efficiently packaged together for shipment to customers, such as stacked with other cartridges 350 in a box. The reservoir 12 has small indentations 302 on either side of the cavity 300, to allow customers to grasp the personal dispenser 20 to remove it from the cavity 300 for use.

The reservoir 12 and personal dispenser 20 are provided with a common label 304 having a first part 306 that is permanently affixed to the outer surface of the reservoir 12, and a second part 308 that is permanently affixed to the outer surface of the personal dispenser 20. The label 304 has a perforated line 310 at the junction between the first part 306 and the second part 308, which allows a user to cleanly tear the label 304 along the perforated line 310 when removing the personal dispenser 20 from the cavity 300. The common label 304 helps to hold the personal dispenser 20 within the cavity 300 during packaging and shipment, and also allows the reservoir 12 and personal dispenser 20 to be marked with required information such as batch numbers and the like. The label 304 also helps customers to detect if the cartridge 350 may have been tampered with during shipment. In particular, by inspecting the cartridge 350 to see if the label 304 has been torn, a customer can detect if the personal dispenser 20 has been removed from the cavity 300.

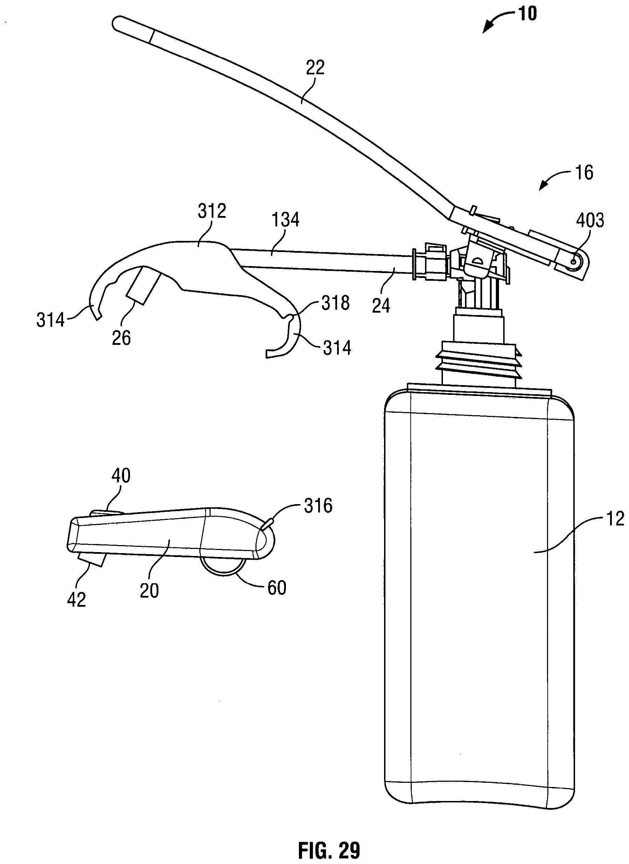

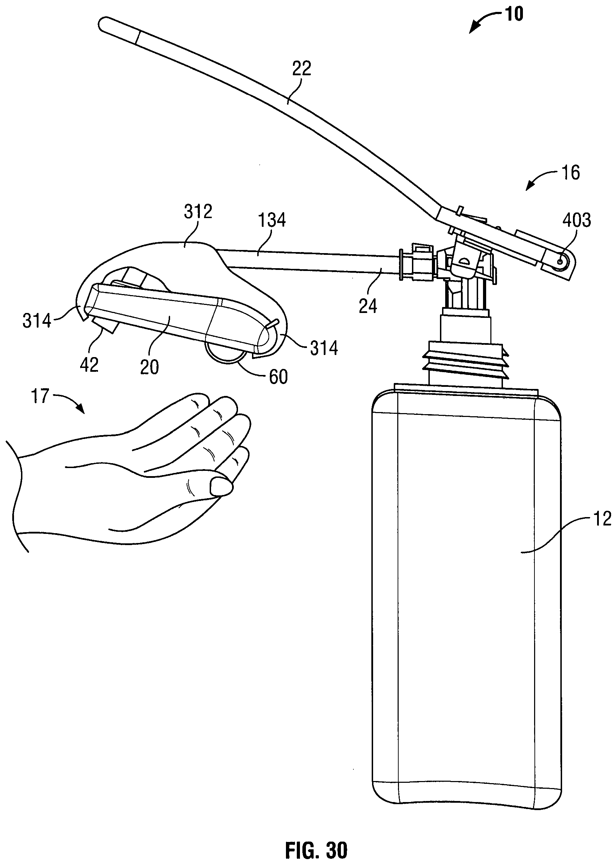

Reference is now made to Figures 29 and 30, which show a liquid master dispenser 10 in accordance with a sixth preferred embodiment of the invention, wherein like reference numerals are used to denote like components. This embodiment of the invention is generally similar to the first embodiment of the invention described above, and operates in a similar manner. The features of the sixth embodiment of the invention which differ from those of the first embodiment are described below.

The sixth embodiment of the invention uses a cradle type dock 312 for coupling the personal dispenser 20 to the spout tube 134 of the master dispenser 10, in place of the mounting dock 28 used in the first embodiment of the invention. The personal dispenser 20 is substantially the same as used in the first embodiment, with exception that the arcuate docking seat 44 is removed. The cradle type dock 312 has resiliently flexible arms 314 that, when in an unbiased state, define an inner profile that is complimentary to the outer profile of the personal dispenser 20. To couple the personal dispenser 20 to the dock 312, the arms 314 are manually pulled apart and the personal dispenser 20 is positioned therebetween, with the outlet end 26 of the spout tube 134 inserted into the inlet port 40 of the personal dispenser 20. The arms 314 are then released, allowing them to return to their unbiased state around the personal dispenser 20, holding the personal dispenser 20 in place. To uncouple the personal dispenser 20 from the dock 312, the arms 314 are pulled apart and the personal dispenser 20 is removed.

Preferably, the personal dispenser 20 and dock 312 incorporate lock-out features that prevent them from coupling with unapproved devices, such as devices made by other manufacturers. For example, as shown in Figures 29 and 30, the personal dispenser 20 can incorporate a rib 316 that fits into a complementary void 318 in the dock 312. When an approved personal dispenser 20 is used with the corresponding dock 312, the rib 316 and the void 318 are aligned so that docking is possible. However, if a user attempts to use an unauthorized personal dispenser 20 having a different shape, including ribs 316 that are not properly aligned with voids 318 in the dock 312, the lock-out features will prevent the dock 312 and the personal dispenser 20 from coupling. This prevents any impairment of performance that might otherwise occur if an unauthorized personal dispenser 20 was coupled to the master dispenser 10. It is to be appreciated that any desired configuration of lock-out features could be used, including, for example, any combination of one or more sets of ribs 316, voids 318, protrusions, cavities, and keyed structures located on the personal dispenser 20 and dock 312, or on other associated structures.

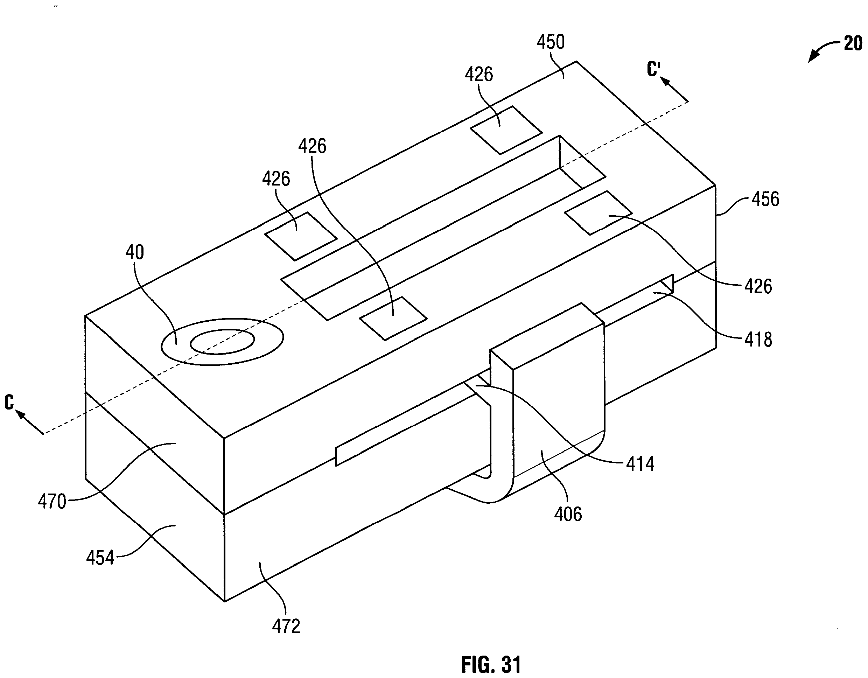

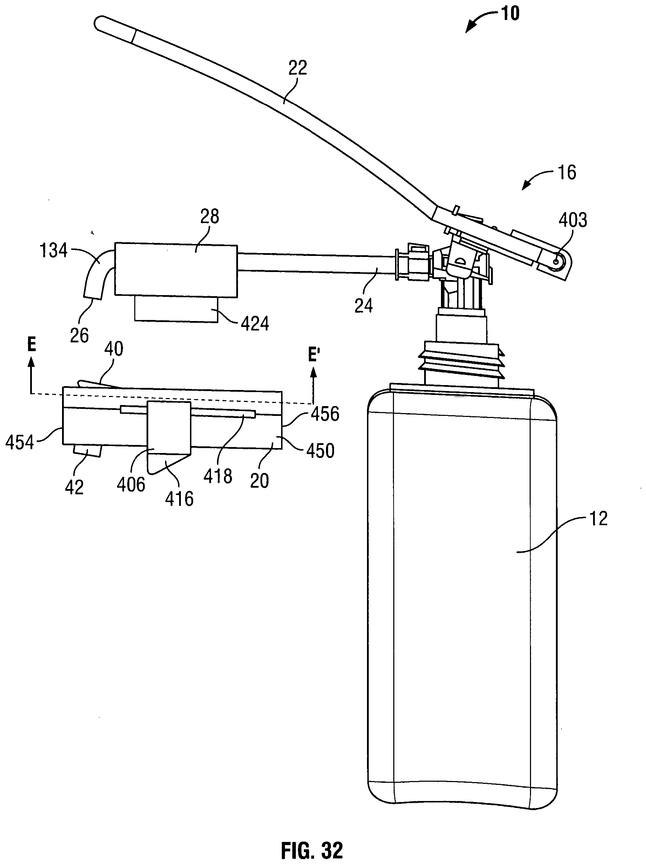

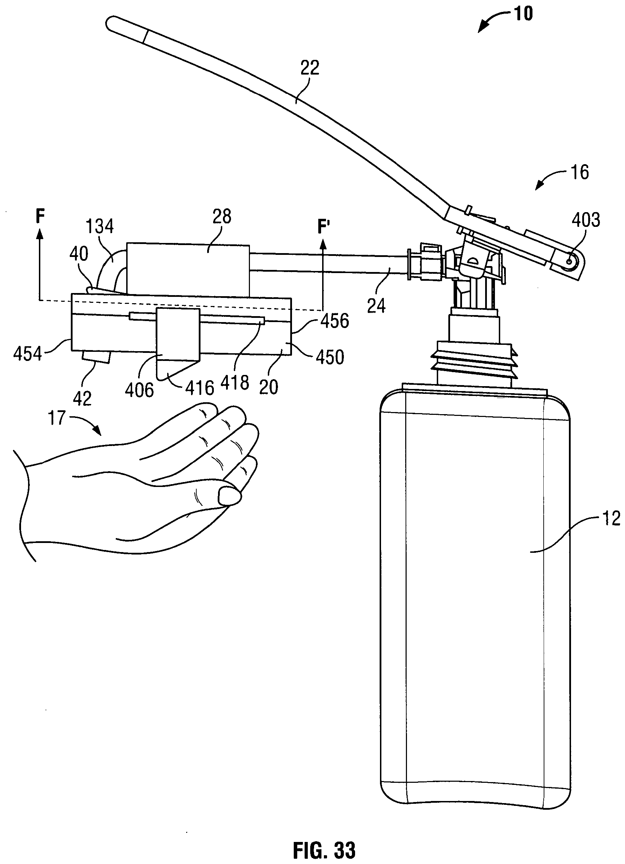

Reference is now made to Figures 31 to 37, which show a liquid master dispenser 10 in accordance with a seventh preferred embodiment of the invention, wherein like reference numerals are used to denote like components. The seventh embodiment of the invention corresponds identically to the first embodiment of the invention described above, with the exception that the personal dispenser 20 has a modified, airless construction. The mounting dock 28 also has an alternate construction for coupling with the modified personal dispenser 20.

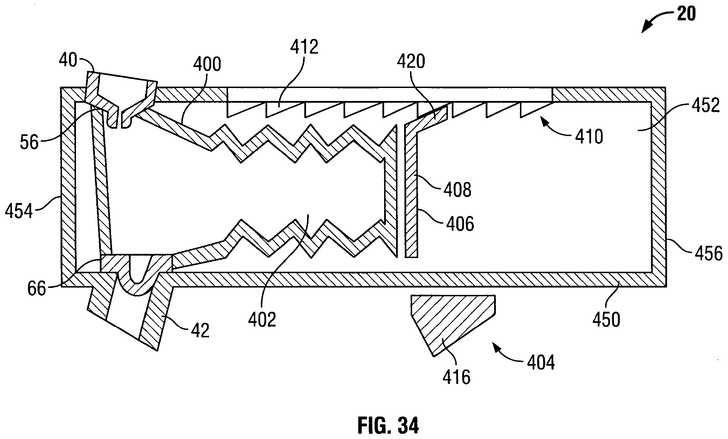

As shown in Figure 31, the personal dispenser 20 has a rigid, generally rectangular outer housing 450. As best shown in Figure 34, the housing 450 defines an internal cavity 452 that extends longitudinally between a first end 454 and a second end 456 of the housing 450. The inlet port 40 and the outlet port 42 are situated near the first end 454, and are spaced from the second end 456. A collapsible internal bellows or bag 400 is arranged within the internal cavity 452, and is attached in a liquid tight manner to the one-way inlet valve 56 of the inlet port 40 and the one-way outlet valve 66 of the outlet port 42. The bag 400defines a liquid chamber 402 for containing liquid within the personal dispenser 20. The bag 400 expands and contracts longitudinally within the cavity 452 as liquid enters from the inlet port 40 or exits from the outlet port 42, such that the volume of the liquid chamber 402 varies depending on the amount of liquid contained therein.

The personal dispenser 20 incorporates a ratcheted plunger mechanism 404 for compressing the bag 400, to thereby expel the liquid contained in the bag 400 out through the outlet port 42. The ratcheted plunger mechanism 404 comprises a plunger body 406 with a flexible pawl 408 that engages with a rack 410 of teeth 412 arranged longitudinally on an internal upper surface of the housing 450. A connecting body 414 connects the flexible pawl 408, located within the housing 450, to an external push button 416. As best shown in Figure 31, the connecting body 414 is slidably arranged within a side slot 418 of the housing 450. The plunger body 406 can be moved longitudinally toward the first end 454 of the housing 450 by manually pushing the push button 416 in that direction.

The flexible pawl 408 has an angled foot 420 that engages with the rack 410. As the plunger body 406 moves toward the first end 454 of the housing 450, the flexible nature of the pawl 408 permits the foot 420 to bend downwards, away from the rack 410, as it passes over the teeth 412. This permits the plunger body 406 to be moved freely toward the first end 454. In doing so, the plunger body 406 presses against the bag 400, causing it to compress. This increases the internal pressure of the liquid within the bag 400, and once a threshold pressure has been reached, forces the liquid through the one-way outlet valve 66 of the outlet port 42. The one-way inlet valve 56 prevents the liquid from discharging from the inlet port 40.

Preferably, the personal dispenser 20 is configured so that each step of the plunger body 406 along the rack 410 consistently discharges a known quantity of the liquid from the outlet port 42. For example, the personal dispenser 20 could be configured to discharge 0.5 ml of the liquid with each step of the plunger body 406 along the rack 410, such that a user is able to estimate the total amount of liquid that has been discharged from the personal dispenser 20 by counting the number of audible clicks that are heard as the plunger 406 is moved. The housing 450 could also be provided with markings that indicate the volume of liquid held within the bag 400 at each position of the push button 416.

Once the bag 400 has been fully compressed and the plunger body 406 is located near the first end 454 of the housing 450, the angle of the foot 420 against the teeth 412 of the rack 410 prevents the foot 420 from being moved back along the rack 410 toward the second end 456. This locks the plunger body 406 in place, preventing the bag 400 from expanding so long as the foot 420 remains engaged with the rack 410. To release the engagement of the foot 420 with the rack 410, and thereby unlock the plunger body 406, the personal dispenser 20 is docked to the mounting dock 28.

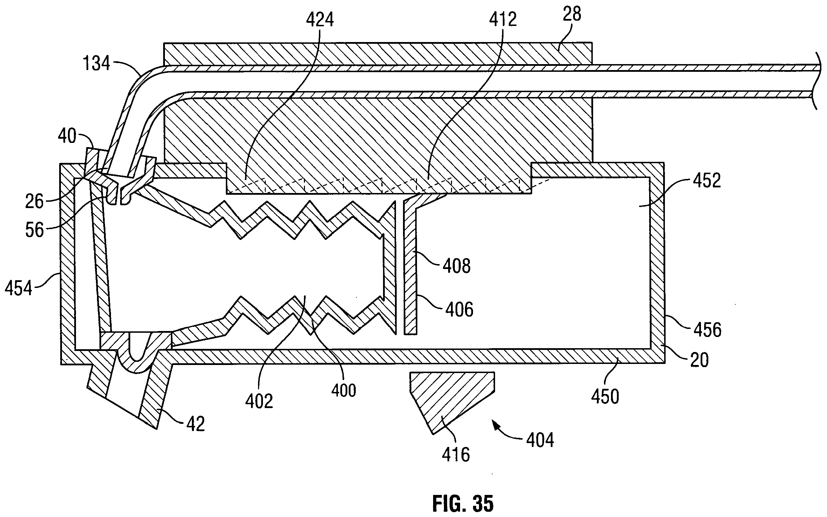

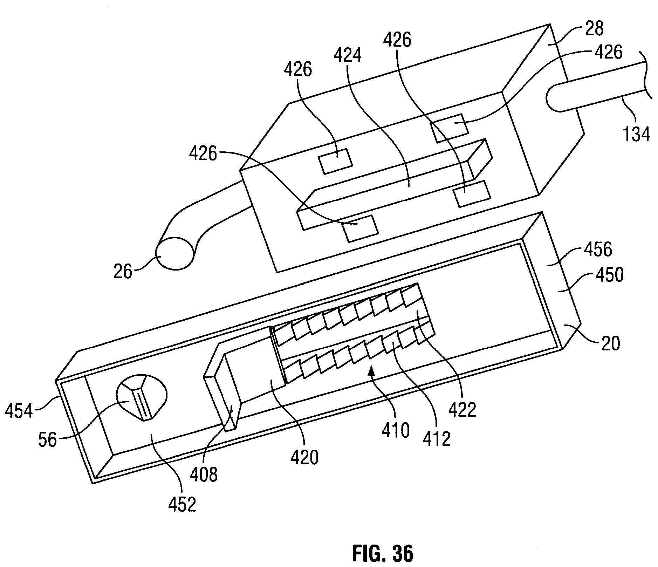

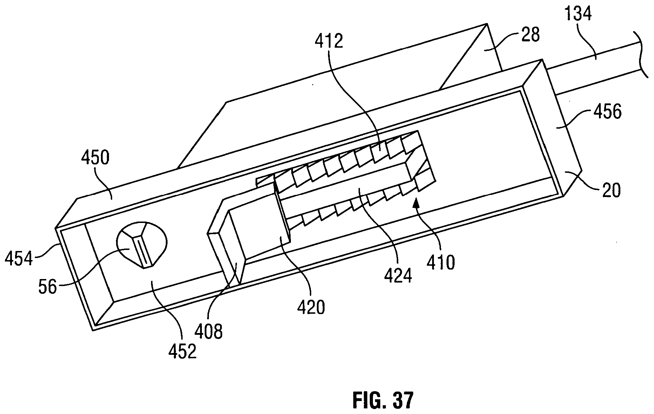

Figures 36 and 37 illustrate the arrangement of the flexible pawl 408 relative to the rack 410 when uncoupled from the mounting dock 28 (Figure 36), and when coupled to the mounting dock 28 (Figure 37). To improve clarity, the bag 400 has been omitted from Figures 36 and 37. As can be seen in Figures 31 and 36, the top surface of the housing 450 has a centrally located mounting slot 422. The teeth 412 of the rack 410 are arranged longitudinally on either side of the mounting slot 422. The slot 422 is sized to receive an unlocking rib 424 of the mounting dock 28 therein. When the personal dispenser 20 is mounted to the mounting dock 28, the unlocking rib 424 is received within the mounting slot 422, and the rib 424 pushes the foot 420 of the flexible pawl 408 away from, and out of engagement with, the rack 410. This is shown in Figures 35 and 37 (in Figure 35, the teeth 412 located behind the rib 424 are shown in dashed lines). With the foot 420 disengaged from the rack 410, the plunger body 406 is unlocked and can freely move toward the second end 456 of the housing 450. As such, it is possible to fill the bag 400 with further liquid while the personal dispenser 20 is mounted to the dock 28.

As shown in Figures 31 and 36, the personal dispenser 20 and the mounting dock 28 have a set of complimentary magnets 426 that align when the unlocking rib 424 of the mounting dock 28 is received within the mounting slot 422 of the personal dispenser 20. These magnets 426 hold the personal dispenser 20 and the mounting dock 28 together when coupled. It is to be appreciated, however, that any other suitable mounting mechanism could be used instead to hold the personal dispenser 20 and the mounting dock 28 together.

As in the previously described embodiments, when the personal dispenser 20 is mounted to the mounting dock 28, the outlet end 26 of the spout tube 134 is sealingly received within the inlet port 40 of the personal dispenser 20. Liquid is pumped into the bag 400 of the personal dispenser 20 by activating the pump assembly 16 of the master dispenser 10, as in the previously described embodiments. The liquid enters the personal dispenser 20 via the inlet port 40, and fills up the bag 400. As the bag 400 fills, it expands longitudinally, pushing the plunger body 406 toward the second end 456 of the housing 450. Once the bag 400 has reached its maximum capacity, any further liquid that is pumped into the bag 400 increases the internal pressure therein. Once the threshold pressure is reached, pumping further liquid into the bag 400 forces an allotment of liquid through the one-way outlet valve 66 of the outlet port 42.

As in the previously described embodiments, the personal dispenser 20 can be removed from the master dispenser 10, and used to provide a supply of the liquid at another location. The liquid within the bag 400 is discharged from the outlet port 42 by pushing the push button 416 toward the first end 454 of the housing 450, in the manner as described above. The personal dispenser 20 can then be refilled as needed by mounting it to the master dispenser 10, and activating the pump assembly 16.

The inventors have appreciated that this construction of the personal dispenser 20 reduces or eliminates contact between the liquid received by the personal dispenser 20 and atmospheric air. In particular, the bag 400 simply expands as it fills with liquid, and there is no air contained therein that needs to be expelled to accommodate the liquid. Furthermore, when the liquid is expelled from the outlet port 42, the bag 400 simply collapses, and there is no need to take in air to replace the discharged liquid.

As such, with this construction of the personal dispenser 20, liquid can be pumped directly from the master dispenser 10 into the personal dispenser 20 without contacting atmospheric air. This reduces the risk of the liquid becoming contaminated by air borne contaminants, including air borne bacteria such as Legionella.

Preferably, the master dispenser 10 is also configured to eliminate contact between the liquid and atmospheric air, to further reduce the risk of contamination. For example, the reservoir 12 is preferably designed to collapse as liquid is discharged therefrom, instead of taking in atmospheric air to replace the discharged liquid. The sealing engagement between the outlet end 26 of the spout tube 134 and the inlet port 40 furthermore prevents the liquid from contacting atmospheric air as it is transferred from the spout tube 134 to the personal dispenser 20.

During manufacturing, the housing 450 of the personal dispenser 20 is optionally constructed from separate top 470 and bottom 472 halves. While the top 470 and bottom 472 halves are detached from one another, the internal cavity 452 is easily accessible so that the internal components of the personal dispenser 20 can be arranged therein. For example, the bag 400 can be placed between the top 470 and bottom 472 halves of the housing 450, so that the bag 400 will sit within the internal cavity 452 once the two halves 470, 472 of the housing 450 are attached together. The plunger body 406 can also be put in place, with the push button 416 arranged on the outer surface of the bottom half 472 of the housing 450, and the connecting body 414 positioned so that it will sit within the side slot 418 once the top half 470 is attached. Once the internal components are in place, the top 470 and bottom 472 halves can be securely attached to one another using glue, heat bonding, snap connections, or any other suitable bonding or attachment techniques.

The reservoir 12 is preferably made from plastic, although any other suitable materials that would adequately contain the liquid could be used instead, such as, in some embodiments of the invention, metal. The liquid is preferably hand soap or hand sanitizer, although other liquids such as hand lotion or sunscreen could be used as desired. Preferably, the reservoir 12 is sized to contain 250 to 5000 ml of the liquid, and more preferably 500 to 2000 ml, although any other desired size could be used instead.

The receptacle 38 is preferably formed from molded plastic, though any other suitable materials could be used instead. Preferably, the receptacle 38 is sized to contain between 10 ml and 80 ml of the liquid, and more preferably between 20 ml and 60 ml, or between 20 ml and 25 ml, although any other desired size could be used. The receptacle 38 may be fully or partially transparent, to permit visual assessment of the volume of the liquid contained therein.

While the preferred embodiments have described the pump assembly 16 as a piston pump, it is to be appreciated that any suitable pump construction could be used including, for example, different positive displacement pumps, gear pumps, plunger pumps, and hydraulic pumps. Furthermore, the invention is not limited to the specific valve constructions that have been depicted and described.

While the preferred embodiments have been described as incorporating particular sensor types, it is to be appreciated that many other sensor types could be used to achieve the desired functionality including, for example, devices that sense mass, motion, temperature, position, electrical properties, magnetic properties, optical properties, or any other detectable properties.

In the first embodiment of the invention described above, preferably the width of the elongated dovetail shaped projection 36 at the front end 32 of the elongated dovetail shaped projection 36 is approximately equal to the width of the elongated dovetail shaped channel 52 at the front end 46 of the elongated dovetail shaped channel 52; and a width of the elongated dovetail shaped projection 36 immediately adjacent to the front end 32 of the elongated dovetail shaped projection 36 is greater than the width of the elongated dovetail shaped channel 52 at the front end 46 of the elongated dovetail shaped channel 52. Also, the width of the elongated dovetail shaped projection 36 at the back end 34 of the elongated dovetail shaped projection 36 is greater than the width of the elongated dovetail shaped channel 52 at the front end of the elongated dovetail shaped channel 52; and the width of the elongated dovetail shaped channel 52 at the back end 48 of the elongated dovetail shaped channel 52 is greater than the width of the elongated dovetail shaped projection 36 at the front end 32 of the elongated dovetail shaped projection 36.

It is to be appreciated that the term "personal dispenser" 20 as used herein is intended to include within its scope mobile dispensing devices that are used by multiple individuals, as well as mobile dispensing devices that may be used by only a single individual. For example, the personal dispenser 20 could be positioned at a point of care, such as at a patient's bedside, where it is available to dispense the liquid to any healthcare worker that visits the patient. The term "personal" is not intended to, in any way, limit the scope of the personal dispenser 20 of the present invention to dispensing devices that are only used by a single user.

As used herein, the term "liquid" is intended to refer broadly to any flowable and relatively dense substance including, for example, gels, creams, foams, and flowable composite materials.

It will be understood that, although various features of the invention have been described with respect to one or another of the embodiments of the invention, the various features and embodiments of the invention may be combined or used in conjunction with other features and embodiments of the invention as described and illustrated herein.

Although this disclosure has described and illustrated certain preferred embodiments of the invention, it is to be understood that the invention is not restricted to these particular embodiments. Rather, the invention is defined by the appended claims.

## Claims

1. A liquid dispenser (10), comprising:
a reservoir (12) for containing liquid to be dispensed;
a dispenser outlet (18) for discharge of the liquid from the reservoir (12);
a discharge mechanism (16) operable to discharge the liquid from the dispenser outlet (18) when activated; and
a removable personal dispenser (20) including a receptacle (38) to receive the liquid via an inlet port (40) and an outlet port (42) for discharge of the liquid from the receptacle (38),
the liquid dispenser (10) is configured to deliver the liquid from the reservoir (12) to the personal dispenser (20),
the personal dispenser (20) is removably coupled to the dispenser outlet (18);
while the personal dispenser (20) is coupled to the dispenser outlet (18), the dispenser outlet (18) sealingly engages with the inlet port (40), placing the dispenser outlet (18) in communication with the receptacle (38);
the discharge mechanism (16) is configured to permit the liquid to be drawn from the reservoir (12) out the dispenser outlet (18) through application of a vacuum pressure to the dispenser outlet (18);
the personal dispenser (20) comprises a dispensing mechanism (60) operable, while the personal dispenser (20) is coupled to the dispenser outlet (18), to create the vacuum pressure to draw the liquid from the reservoir (12), out the dispenser outlet (18), and into the receptacle (38); and
(a) while the personal dispenser (20) is coupled to the dispenser outlet (18), the liquid discharged from the dispenser outlet (18) accumulates in the receptacle (38) until the receptacle (38) contains a preselected volume of the liquid, and once the receptacle (38) contains the preselected volume of the liquid, the discharge of further said liquid from the dispenser outlet (18) causes the liquid contained in the receptacle (38) to be discharged from the outlet port (42); and
(b) while the personal dispenser (20) is removed from the dispenser outlet (18):
(i) the personal dispenser (20) is configured to be carried manually to locations remote from the dispenser outlet (18) for selectively discharging the liquid contained in the receptacle (38) from the outlet port (42); and
(ii) the discharge mechanism (16) is operable to discharge the liquid contained in the reservoir (12) from the dispenser outlet (18).

2. The liquid dispenser (10) according to claim 1, wherein
the dispensing mechanism (60) is manually operative to create the vacuum pressure.

3. The liquid dispenser (10) according to claim 1 or 2, wherein
the discharge mechanism (16) requires electrical power for operation,
while the personal dispenser (20) is coupled to the dispenser outlet (18), if the electrical power required for operation of the discharge mechanism (16) is not available, operation of the dispensing mechanism (60) draws the liquid from the reservoir (12), out the dispenser outlet (18), into the receptacle (38).

4. The liquid dispenser (10) according to any one of claims 1 to 3, wherein
the personal dispenser (20) includes the inlet port (40) for receiving the liquid discharged from the dispenser outlet (18) and communicating the liquid to the receptacle (38).

5. The liquid dispenser (10) according to any one of claims 1 to 4,
wherein the inlet port (40) comprises a one-way inlet valve (56) that permits fluid to enter the receptacle (38) through the inlet port (40), and prevents fluid from exiting the receptacle (38) through the inlet port (40);
wherein the outlet port (42) comprises a one-way outlet valve (66) that permits fluid to exit the receptacle (38) through the outlet port (42), and prevents fluid from entering the receptacle (38) through the outlet port (42); and
wherein the receptacle (38) defines an internal volume and is compressible to selectively reduce the internal volume from an uncompressed volume to a compressed volume upon application of a compressing force, to thereby discharge the liquid from the outlet port (42).

6. The liquid dispenser (10) according to any one of claims 1 to 5, **characterized in that** the receptacle (38) comprises:
an internal chamber (68) having an open end (74) for receiving and expelling the liquid therefrom; and
a manually compressible diaphragm (60) defining one side of the internal chamber (68);
wherein the personal dispenser (20) is configured so that compression of the diaphragm (60) effects the discharge of the liquid from the outlet port (42);
wherein the internal chamber (68) is configured so that, while the personal dispenser (20) is coupled to the dispenser outlet (18), the open end (74) is positioned at a designated height above the diaphragm (60), the designated height corresponding to a level of the liquid within the receptacle (38) when the receptacle (38) contains a designated volume of the liquid, so that the liquid only enters the internal chamber (68) once the receptacle (38) contains the designated volume of the liquid;
wherein the diaphragm (60) is at least partially transparent and is visible to a user to provide a visual indication of whether the internal chamber (68) contains the liquid; and
wherein the receptacle (38) further comprises a baffle (62) interposed between the inlet port (40) and the outlet port (42), for directing the liquid received by the inlet port (40) toward the internal chamber (68).

7. The liquid dispenser (10) according to any one of claims 1 to 6, **characterized in that** the outlet port (42) comprises an opening (78) in fluid communication with the receptacle (38);
wherein the personal dispenser (20) is configured so that, while the personal dispenser (20) is coupled to the dispenser outlet (18), the opening (78) is positioned at a preselected height relative to the receptacle (38), the preselected height corresponding to a level of the liquid within the receptacle (38) when the receptacle (38) contains the preselected volume of the liquid;
wherein while the personal dispenser (20) is coupled to the dispenser outlet (18), while the level of the liquid within the receptacle (38) is below the preselected height, the liquid discharged into the receptacle (38) from the dispenser outlet (18) displaces air in the receptacle (38) out the outlet port (42).

8. The liquid dispenser (10) according to any one of claims 1 to 7, **characterized in that** the liquid dispenser (10) further comprises:
a sensor (82) configured to detect if the personal dispenser (20) is coupled to the dispenser outlet (18);
a sensor (84) configured to detect if the receptacle (38) contains the preselected volume of the liquid; and
a control system (86) that receives information from the sensors (82, 84) and controls the discharge mechanism (16);
wherein the control system (86) is configured to, upon detecting that the personal dispenser (20) has been coupled to the dispenser outlet (18), activate the discharge mechanism (16) to discharge the liquid to fill the receptacle (38) when detecting that the receptacle (38) does not contain the preselected volume of the liquid, and to end the activation of the discharge mechanism (16) to fill the receptacle (38) upon detecting that the receptacle (38) contains the preselected volume of the liquid.

9. The liquid dispenser (10) according to any one of claims 1 to 8, **characterized in that** the reservoir (12) has a cavity (300) sized for removably receiving the personal dispenser (20);
wherein, prior to assembly of the liquid dispenser (10), the personal dispenser (20) is held within the cavity (300) of the reservoir (12) in an initial bundled configuration;
the liquid dispenser (10) further comprising a label (304) that is secured to a face of the reservoir (12) and a face of the personal dispenser (20) while in the initial bundled configuration;
wherein the label (304) is configured to be severed upon removal of the personal dispenser (20) from the cavity (300), so as to leave a first portion (306) of the label (304) secured to the face of the reservoir (12) and a second portion (308) of the label (304) secured to the face of the personal dispenser (20).

10. The liquid dispenser (10) according to any one of claims 1 to 9, wherein the liquid comprises a hand cleaning liquid, and the hand cleaning liquid comprises a hand soap or a hand sanitizer.

## Patentansprüche

1. Flüssigkeitsspender (10), umfassend:
ein Reservoir (12) zur Aufnahme der abzugebenden Flüssigkeit;
einen Spenderauslass (18) zum Abgeben der Flüssigkeit aus dem Reservoir (12);
einen Abgabemechanismus (16), der betätigbar ist, um die Flüssigkeit aus dem Spenderauslass (18) abzugeben, wenn er aktiviert wird; und
einen abnehmbaren persönlichen Spender (20), umfassend einen Behälter (38) zur Aufnahme der Flüssigkeit über eine Einlassöffnung (40) und eine Auslassöffnung (42) zur Abgabe der Flüssigkeit aus dem Behälter (38),
der Flüssigkeitsspender (10) so konfiguriert ist, dass er die Flüssigkeit aus dem Reservoir (12) an den persönlichen Spender (20) abgibt,
der persönliche Spender (20) abnehmbar mit dem Spenderauslass (18) verbunden ist;
während der persönliche Spender (20) mit dem Spenderauslass (18) gekoppelt ist, der Spenderauslass (18) abdichtend mit der Einlassöffnung (40) in Eingriff steht, wodurch der Spenderauslass (18) in Verbindung mit dem Behälter (38) gebracht wird;
der Abgabemechanismus (16) so konfiguriert ist, dass er es ermöglicht, die Flüssigkeit von dem Reservoir (12) aus dem Spenderauslass (18) durch Anlegen eines Unterdrucks an den Spenderauslass (18) zu ziehen;
der persönliche Spender (20) einen Abgabemechanismus (60) umfasst, der betätigbar ist, während der persönliche Spender (20) mit dem Spenderauslass (18) gekoppelt ist, um den Unterdruck zu erzeugen, um die Flüssigkeit von dem Reservoir (12) aus dem Spenderauslass (18) und in den Behälter (38) zu ziehen; und
(a) während der persönliche Spender (20) mit dem Spenderauslass (18) gekoppelt ist, sich die aus dem Spenderauslass (18) abgegebene Flüssigkeit in dem Behälter (38) ansammelt, bis der Behälter (38) ein vorgewähltes Volumen der Flüssigkeit enthält, und sobald der Behälter (38) das vorgewählte Volumen der Flüssigkeit enthält, die Abgabe weiterer Flüssigkeit aus dem Spenderauslass (18) bewirkt, dass die in dem Behälter (38) enthaltene Flüssigkeit aus der Auslassöffnung (42) abgegeben wird; und
(b) während der persönliche Spender (20) von dem Spenderauslass (18) entfernt wird:
(i) der persönliche Spender (20) so konfiguriert ist, dass er manuell zu Orten getragen werden kann, die von dem Spenderauslass (18) entfernt sind, um die in dem Behälter (38) enthaltene Flüssigkeit aus der Auslassöffnung (42) selektiv abzugeben; und
(ii) der Abgabemechanismus (16) betätigbar ist, um die in dem Behälter (12) enthaltene Flüssigkeit aus dem Spenderauslass (18) abzugeben.

2. Flüssigkeitsspender (10) nach Anspruch 1, wobei
der Ausgabemechanismus (60) manuell betätigt wird, um den Unterdruck zu erzeugen.

3. Flüssigkeitsabgabevorrichtung (10) nach Anspruch 1 oder 2, wobei
der Abgabemechanismus (16) für den Betrieb elektrische Energie erfordert,
während der persönliche Spender (20) mit dem Spenderauslass (18) gekoppelt ist, wenn die für den Betrieb des Abgabemechanismus (16) erforderliche elektrische Energie nicht verfügbar ist, der Betrieb des Abgabemechanismus (60) die Flüssigkeit von dem Reservoir (12) aus dem Spenderauslass (18) in den Behälter (38) zieht.

4. Flüssigkeitsspender (10) nach einem der Ansprüche 1 bis 3, wobei
der persönliche Spender (20) die Einlassöffnung (40) zur Aufnahme der aus dem Spenderauslass (18) abgegebenen Flüssigkeit und zur Weiterleitung der Flüssigkeit an den Behälter (38) umfasst.

5. Flüssigkeitsspender (10) nach einem der Ansprüche 1 bis 4,
wobei die Einlassöffnung (40) ein Einweg-Einlassventil (56) umfasst, das den Eintritt von Fluid in den Behälter (38) durch die Einlassöffnung (40) ermöglicht und verhindert, dass Fluid den Behälter (38) durch die Einlassöffnung (40) verlässt;
wobei die Auslassöffnung (42) ein Einweg-Auslassventil (66) umfasst, das den Austritt von Fluid aus dem Behälter (38) durch die Auslassöffnung (42) ermöglicht und den Eintritt von Fluid in den Behälter (38) durch die Auslassöffnung (42) verhindert; und
wobei der Behälter (38) ein Innenvolumen definiert und komprimierbar ist, um das Innenvolumen selektiv von einem nicht komprimierten Volumen auf ein komprimiertes Volumen bei Anwendung einer Kompressionskraft zu reduzieren, um dadurch die Flüssigkeit aus der Auslassöffnung (42) abzugeben.

6. Flüssigkeitsspender (10) nach einem der Ansprüche 1 bis 5, **dadurch gekennzeichnet, dass** der Behälter (38) umfasst:
eine Innenkammer (68) mit einem offenen Ende (74) zum Aufnehmen und Ausstoßen der Flüssigkeit; und
eine manuell zusammendrückbare Membran (60), die eine Seite der Innenkammer (68) definiert;
wobei der persönliche Spender (20) so konfiguriert ist, dass das Zusammendrücken der Membran (60) die Abgabe der Flüssigkeit aus der Auslassöffnung (42) bewirkt;
wobei die Innenkammer (68) so konfiguriert ist, dass, während der persönliche Spender (20) mit dem Spenderauslass (18) verbunden ist, das offene Ende (74) in einer bestimmten Höhe über der Membran (60) positioniert ist, wobei die bestimmte Höhe einem Pegel der Flüssigkeit innerhalb des Behälters (38) entspricht, wenn der Behälter (38) ein bestimmtes Volumen der Flüssigkeit enthält, so dass die Flüssigkeit nur in die Innenkammer (68) eintritt, wenn der Behälter (38) das bestimmte Volumen der Flüssigkeit enthält;
wobei die Membran (60) zumindest teilweise transparent ist und für einen Benutzer sichtbar ist, um eine visuelle Anzeige zu liefern, ob die Innenkammer (68) die Flüssigkeit enthält; und
wobei der Behälter (38) ferner ein Ablenkblech (62) umfasst, das zwischen der Einlassöffnung (40) und der Auslassöffnung (42) angeordnet ist, um die von der Einlassöffnung (40) aufgenommene Flüssigkeit in die Innenkammer (68) zu leiten.

7. Flüssigkeitsspender (10) nach einem der Ansprüche 1 bis 6, **dadurch gekennzeichnet, dass** die Auslassöffnung (42) eine Öffnung (78) aufweist, die in Fluidverbindung mit dem Behälter (38) steht;
wobei der persönliche Spender (20) so konfiguriert ist, dass, während der persönliche Spender (20) mit dem Spenderauslass (18) gekoppelt ist, die Öffnung (78) in einer vorgewählten Höhe relativ zu dem Behälter (38) positioniert ist, wobei die vorgewählte Höhe einem Pegel der Flüssigkeit innerhalb des Behälters (38) entspricht, wenn der Behälter (38) das vorgewählte Volumen der Flüssigkeit enthält;
wobei, während der persönliche Spender (20) mit dem Spenderauslass (18) gekoppelt ist, während der Pegel der Flüssigkeit innerhalb des Behälters (38) unter der vorgewählten Höhe liegt, die in den Behälter (38) aus dem Spenderauslass (18) abgegebene Flüssigkeit die Luft im Behälter (38) aus der Auslassöffnung (42) verdrängt.

8. Flüssigkeitsspender (10) nach einem der Ansprüche 1 bis 7, **dadurch gekennzeichnet, dass** der Flüssigkeitsspender (10) ferner umfasst:
einen Sensor (82), der so konfiguriert ist, dass er erkennt, ob der persönliche Spender (20) mit dem Spenderauslass (18) verbunden ist;
einen Sensor (84), der so konfiguriert ist, dass er erfasst, ob der Behälter (38) das vorgewählte Volumen der Flüssigkeit enthält; und
ein Steuersystem (86), das Informationen von den Sensoren (82, 84) empfängt und den Abgabemechanismus (16) steuert;
wobei das Steuersystem (86) so konfiguriert ist, dass es beim Erfassen, dass der persönliche Spender (20) mit dem Spenderauslass (18) gekoppelt wurde, den Abgabemechanismus (16) aktiviert, um die Flüssigkeit abzugeben, um den Behälter (38) zu füllen, wenn erfasst wird, dass der Behälter (38) nicht das vorgewählte Volumen der Flüssigkeit enthält, und um die Aktivierung des Abgabemechanismus (16) zu beenden, um den Behälter (38) zu füllen, wenn erfasst wird, dass der Behälter (38) das vorgewählte Volumen der Flüssigkeit enthält.

9. Flüssigkeitsspender (10) nach einem der Ansprüche 1 bis 8, **dadurch gekennzeichnet, dass** der Behälter (12) einen Hohlraum (300) aufweist, der so bemessen ist, dass er den persönlichen Spender (20) herausnehmbar aufnehmen kann;
wobei vor dem Zusammenbau des Flüssigkeitsspenders (10) der persönliche Spender (20) innerhalb des Hohlraums (300) des Behälters (12) in einer anfänglich gebündelten Konfiguration gehalten wird;
wobei der Flüssigkeitsspender (10) femer ein Etikett (304) umfasst, das an einer Fläche des Behälters (12) und einer Fläche des persönlichen Spenders (20) befestigt ist, während er sich in der anfänglich gebündelten Konfiguration befindet;
wobei das Etikett (304) so konfiguriert ist, dass es beim Entfernen des persönlichen Spenders (20) aus dem Hohlraum (300) abgetrennt wird, so dass ein erster Abschnitt (306) des Etiketts (304) an der Fläche des Behälters (12) befestigt bleibt und ein zweiter Abschnitt (308) des Etiketts (304) an der Fläche des persönlichen Spenders (20) befestigt bleibt.

10. Flüssigkeitsspender (10) nach einem der Ansprüche 1 bis 9, wobei die Flüssigkeit eine Handreinigungsflüssigkeit umfasst, und die Handreinigungsflüssigkeit eine Handseife oder ein Handdesinfektionsmittel umfasst.

## Revendications

1. Distributeur de liquide (10), comprenant :
un réservoir (12) pour contenir un liquide à distribuer ;
un orifice de sortie de distributeur (18) pour l'évacuation du liquide du réservoir (12) ;
un mécanisme d'évacuation (16) pouvant fonctionner pour évacuer le liquide de l'orifice de sortie de distributeur (18) lorsqu'il est activé ; et
un distributeur individuel amovible (20) comprenant un réceptacle (38) pour recevoir le liquide par l'intermédiaire d'un orifice d'entrée (40) et un orifice de sortie (42) pour l'évacuation du liquide du réceptacle (38),
le distributeur de liquide (10) étant conçu pour décharger le liquide du réservoir (12) vers le distributeur individuel (20),
le distributeur individuel (20) étant accouplé de manière amovible à l'orifice de sortie de distributeur (18) ;
tandis que le distributeur individuel (20) est accouplé à l'orifice de sortie de distributeur (18), l'orifice de sortie de distributeur (18) s'engage de manière étanche avec l'orifice d'entrée (40), plaçant l'orifice de sortie de distributeur (18) en communication avec le réceptacle (38) ;
le mécanisme d'évacuation (16) étant conçu pour permettre au liquide d'être extrait du réservoir (12) hors de l'orifice de sortie de distributeur (18) par l'application d'une pression de vide à l'orifice de sortie de distributeur (18) ;
le distributeur individuel (20) comprenant un mécanisme de distribution (60) pouvant fonctionner, tandis que le distributeur individuel (20) est accouplé à l'orifice de sortie de distributeur (18), afin de créer la pression de vide pour extraire le liquide du réservoir (12), hors de l'orifice de sortie de distributeur (18), et dans le réceptacle (38) ; et
(a) tandis que le distributeur individuel (20) est accouplé à l'orifice de sortie de distributeur (18), le liquide évacué de l'orifice de sortie de distributeur (18) s'accumule dans le réceptacle (38) jusqu'à ce que le réceptacle (38) contienne un volume présélectionné du liquide, et une fois que le réceptacle (38) contient le volume présélectionné du liquide, l'évacuation dudit liquide supplémentaire de l'orifice de sortie de distributeur (18) conduisant à l'évacuation du liquide contenu dans le réceptacle (38) de l'orifice de sortie (42) ; et
(b) tandis que le distributeur individuel (20) est retiré de l'orifice de sortie de distributeur (18) :
(i) le distributeur individuel (20) est conçu pour être transporté manuellement vers des emplacements distants de l'orifice de sortie de distributeur (18) pour évacuer sélectivement le liquide contenu dans le réceptacle (38) depuis l'orifice de sortie (42) ; et
(ii) le mécanisme d'évacuation (16) pouvant fonctionner pour évacuer le liquide contenu dans le réservoir (12) depuis l'orifice de sortie de distributeur (18).

2. Distributeur de liquide (10) selon la revendication 1,
le mécanisme de distribution (60) pouvant manuellement fonctionner pour créer la pression de vide.

3. Distributeur de liquide (10) selon la revendication 1 ou 2,
le mécanisme d'évacuation (16) nécessitant de l'énergie électrique pour le fonctionnement,
tandis que le distributeur individuel (20) est accouplé à l'orifice de sortie de distributeur (18), si l'énergie électrique requise pour le fonctionnement du mécanisme d'évacuation (16) n'est pas disponible, le fonctionnement du mécanisme de distribution (60) extrait le liquide du réservoir (12), hors de l'orifice de sortie de distributeur (18), dans le réceptacle (38).

4. Distributeur de liquide (10) selon l'une quelconque des revendications 1 à 3,
le distributeur individuel (20) comprenant l'orifice d'entrée (40) pour recevoir le liquide évacué de l'orifice de sortie de distributeur (18) et communiquant le liquide au réceptacle (38).

5. Distributeur de liquide (10) selon l'une quelconque des revendications 1 à 4,
l'orifice d'entrée (40) comprenant une soupape d'entrée à une voie (56) qui permet au fluide de pénétrer dans le réceptacle (38) à travers l'orifice d'entrée (40), et qui empêche le fluide de sortir du réceptacle (38) à travers l'orifice d'entrée (40) ;
l'orifice de sortie (42) comprenant une soupape de sortie à une voie (66) qui permet au fluide de sortir du réceptacle (38) à travers l'orifice de sortie (42), et qui empêche le fluide de pénétrer dans le réceptacle (38) à travers l'orifice de sortie (42) ; et
le réceptacle (38) définissant un volume interne et pouvant être comprimé pour réduire sélectivement le volume interne depuis un volume non comprimé vers un volume comprimé lors de l'application d'une force de compression, pour évacuer ainsi le liquide de l'orifice de sortie (42) .

6. Distributeur de liquide (10) selon l'une quelconque des revendications 1 à 5, **caractérisé en ce que** le réceptacle (38) comprend :
une chambre interne (68) ayant une extrémité ouverte (74) pour recevoir et expulser le liquide de celle-ci ; et
un diaphragme (60) pouvant être manuellement comprimé définissant un côté de la chambre interne (68) ;
le distributeur individuel (20) étant conçu de sorte qu'une compression du diaphragme (60) effectue l'évacuation du liquide de l'orifice de sortie (42) ;
la chambre interne (68) étant conçue de sorte que, tandis que le distributeur individuel (20) est accouplé à l'orifice de sortie de distributeur (18), l'extrémité ouverte (74) est positionnée au niveau d'une hauteur désignée au-dessus du diaphragme (60), la hauteur désignée correspondant à un niveau du liquide à l'intérieur du réceptacle (38) lorsque le réceptacle (38) contient un volume désigné du liquide, de sorte que le liquide pénètre uniquement dans la chambre interne (68) une fois que le réceptacle (38) contient le volume désigné du liquide ;
le diaphragme (60) étant au moins partiellement transparent et étant visible à un utilisateur pour fournir une indication visuelle du fait que la chambre interne (68) contient le liquide ; et
le réceptacle (38) comprenant en outre une chicane (62) interposée entre l'orifice d'entrée (40) et l'orifice de sortie (42), pour diriger le liquide reçu par l'orifice d'entrée (40) vers la chambre interne (68).

7. Distributeur de liquide (10) selon l'une quelconque des revendications 1 à 6, **caractérisé en ce que** l'orifice de sortie (42) comprend une ouverture (78) en communication fluidique avec le réceptacle (38) ;
le distributeur individuel (20) étant conçu de sorte que, tandis que le distributeur individuel (20) est accouplé à l'orifice de sortie de distributeur (18), l'ouverture (78) est positionnée au niveau d'une hauteur présélectionnée par rapport au réceptacle (38), la hauteur présélectionnée correspondant à un niveau du liquide à l'intérieur du réceptacle (38) lorsque le réceptacle (38) contient le volume présélectionné du liquide ;
tandis que le distributeur individuel (20) est accouplé à l'orifice de sortie de distributeur (18), tandis que le niveau du liquide à l'intérieur du réceptacle (38) est inférieur à la hauteur présélectionnée, le liquide évacué dans le réceptacle (38) depuis l'orifice de sortie de distributeur (18) déplace l'air dans le réceptacle (38) hors de l'orifice de sortie (42).

8. Distributeur de liquide (10) selon l'une quelconque des revendications 1 à 7, **caractérisé en ce que** le distributeur de liquide (10) comprend en outre :
un capteur (82) conçu pour détecter si le distributeur individuel (20) est accouplé à l'orifice de sortie de distributeur (18) ;
un capteur (84) conçu pour détecter si le réceptacle (38) contient le volume présélectionné du liquide ; et
un système de commande (86) qui reçoit des informations des capteurs (82, 84) et qui commande le mécanisme d'évacuation (16) ;
le système de commande (86) étant conçu pour, lors de la détection que le distributeur individuel (20) a été accouplé à l'orifice de sortie de distributeur (18), activer le mécanisme d'évacuation (16) pour évacuer le liquide pour remplir le réceptacle (38) lors de la détection que le réceptacle (38) ne contient pas le volume présélectionné du liquide, et pour achever l'activation du mécanisme d'évacuation (16) pour remplir le réceptacle (38) lors de la détection que le réceptacle (38) contient le volume présélectionné du liquide.

9. Distributeur de liquide (10) selon l'une quelconque des revendications 1 à 8, **caractérisé en ce que** le réservoir (12) présente une cavité (300) calibrée pour recevoir de manière amovible le distributeur individuel (20) ;
avant l'assemblage du distributeur de liquide (10), le distributeur individuel (20) est maintenu à l'intérieur de la cavité (300) du réservoir (12) en une configuration initiale en faisceau;
le distributeur de liquide (10) comprenant en outre une étiquette (304) qui est solidement fixée à une face du réservoir (12) et une face du distributeur individuel (20) tandis qu'il se trouve dans la configuration initiale en faisceau;
l'étiquette (304) étant conçue pour être déchirée lors du retrait du distributeur individuel (20) de la cavité (300), afin de laisser une première portion (306) de l'étiquette (304) solidement fixée à la face du réservoir (12) et une seconde portion (308) de l'étiquette (304) solidement fixée à la face du distributeur individuel (20).

10. Distributeur de liquide (10) selon l'une quelconque des revendications 1 à 9, le liquide comprenant un liquide de nettoyage des mains, et le liquide de nettoyage des mains comprenant un savon pour les mains ou un agent d'hygiène pour les mains.
